(19) Europäisches Patentamt
European Patent Office
Office européen des brevets

(11) **EP 4 717 781 A2**

(12) **EUROPEAN PATENT APPLICATION**

(43) Date of publication:
**01.04.2026 Bulletin 2026/14**

(21) Application number: **26155399.4**

(22) Date of filing: **20.10.2023**

(51) International Patent Classification (IPC):
**C12Q 1/6806** (2018.01)

(52) Cooperative Patent Classification (CPC):
(C-Sets available)
**G16B 20/50; C12Q 1/6806; G16B 20/00;
G16B 20/20; G16B 40/00** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(62) Document number(s) of the earlier application(s) in
accordance with Art. 76 EPC:
**23851091.1 / 4 569 133**

(71) Applicants:
• **Beijing Changping Laboratory
Changping District
Beijing 102206 (CN)**
• **Peking University
Beijing 100871 (CN)**

(72) Inventors:
• **HUANG, Lei
Beijing 100871 (CN)**

• **GE, Hao
Beijing 100871 (CN)**
• **ZHANG, Ruiqi
Beijing 100871 (CN)**
• **XIE, Xiaoliang
Beijing 102206 (CN)**

(74) Representative: **Metroconsult Srl
Via Sestriere, 100
10060 None (TO) (IT)**

Remarks:
•The complete document including Reference
Table(s) and the Sequence Listing(s) can be
downloaded from the EPO website
•This application was filed on 30-01-2026 as a
divisional application to the application mentioned
under INID code 62.

(54) **METHODS FOR RELIABLE NONINVASIVE PREIMPLANTATION GENETIC TESTING**

(57) The present invention provides one or more accurate methods for noninvasive preimplantation genetic testing. Specifically, the present invention provides systems and methods for analyzing a biological sample of culture medium from an in vitro cultured embryo to determine the susceptibility of a genetic disease in the embryo.

Fig. 1

(52) Cooperative Patent Classification (CPC): (Cont.)

C-Sets
**C12Q 1/6806, C12Q 2535/122, C12Q 2531/101**

**Description**

**FIELD OF THE INVENTION**

The present invention relates to one or more accurate methods for noninvasive preimplantation genetic testing. Specifically, the present invention relates to systems and methods for amplifying minute amounts of DNA in a solution, and linkage analysis methods of analyzing a biological sample of culture medium from an in vitro cultured embryo to determine the susceptibility of a genetic disease in the embryo.

**BACKGROUND OF THE INVENTION**

Preimplantation genetic testing (PGT) (Handyside et al., 1989 and 1990 for first children born) as in known in the art has been widely used for clinical *in vitro* fertilization (IVF) to avoid selecting embryos with monogenic diseases (Kerem et al., 1989; Handyside et al., 1992; Liu et al., 1994; Harton et al., 1996; Ao et al., 1998; Sermon et al., 1998; Xu et al., 1999; Hussey et al., 1999; Ray et al., 2000; De Rycke et al., 2001; Moutou et al., 2001; Verlinsky et al., 2001; Sermon et al., 2001; Girardet et al., 2003; Fiorentino et al., 2006; Kahraman et al., 2014), aneuploidy (Munné et al., 1993; Wilton et al., 2001; Wells et al., 2002; Treff et al., 2010; Gutiérrez-Mateo et al., 2011; Yang et al., 2012; Scott et al., 2013; Forman et al., 2013; Wells et al., 2014; Rubio et al., 2017 ), structure variation(Conn et al., 1998; Scriven et al., 1998; Coonen et al 2000; Munné et al., 2000; Escudero et al., 2003; Melotte et al., 2004; Le Caignec et al., 2006; Traversa et al., 2010; Fiorentino et al., 2010; Fiorentino et al., 2010; Rius et al., 2011; Fiorentino et al., 2011; Treff et al., 2016; Zhang et al., 2017; Tan et al., 2013; Chow et al., 2018), or polygenic disease (Treff et al., 2019; Kumar et al. 2022). It has been reported that using one NGS-based testing, chromosome copy number analysis and linkage analysis and targeted haplotyping for mutation can be done simultaneously (Yan et al., 2015). Currently for PGT, a trophectoderm (TE) biopsy is needed (Dokras et al., 1990; Handyside et al., 1990; Kokkali et al., 2005), which is invasive and subject to sampling bias.

Use of the cell-free DNA in blastocoele fluid (BF) for amplification and genetic testing was reported as a minimally invasive preimplantation genetic testing (Palini 2013, Gianaroli et al., 2014; Tobler et al., 2015; Magli et al., 2016; Zhang et al., 2016; Shangguan et al., 2017;Capalbo et al., 2018; Tsuiko et al., 2018; Magli et al., 2018).

A completely noninvasive way for preimplantation genetic testing is described for aneuploidy and for monogenic disease using the cell-free DNA in spent embryo culture medium (Galluzzi et al.,2015; Wu et al., 2015; Xu et al., 2016, Shamonki et al., 2016; Feichtinger et al., 2017; Lane et al., 2017; Liu et al., 2017; Ho et al., 2018; Vera-Rodriguez et al., 2018; Capalbo et al., 2018; Fang et al., 2019; Huang et al., 2019; Rubio et al., 2019; Yeung et al., 2019; Jiao et al. 2019; Ou et al., 2022 ).

Minimally invasive preimplantation genetic testing and non-invasive preimplantation genetic testing are the two major methods for avoiding biopsy damage in preimplantation genetic testing. The method of embryo culture and the approach of spent culture medium (SCM) collection, as well as the single-cell whole genome amplification (scWGA) method used for minute amounts of cell-free DNA, can directly affect the results. The success rate of amplification of samples from blastocoele fluid and spent embryo culture medium varies greatly in different clinical centers. When using the same amplification method, the success rate of amplification in BF was much lower than that in spent culture medium (SCM), such as 62.5% SCM vs 44.4% BF reported by Galluzzi et al.

(2015), 89.7% SCM vs 27.4% BF reported by Capalbo et al. (2018). Although the amplification success rate of SCM was higher than that of BF, the results of SCM were affected by maternal contamination and by the addition of exogenous proteins to the culture medium for embryo development. It has been shown that maternal DNA fragments in maternal blood are longer than fetal DNA fragments (Chan et al., 2004) and the bias of the amplification technique for fragment length makes it ineffective for fetal DNA amplification. In addition, it is important to ensure the accuracy of amplification while maintaining the yield for clinical testing use. Currently, available commercial single-cell amplification kits are based on DOP-PCR, MDA or MALBAC techniques, and these commercial kits differ in amplification efficiency and fidelity (Huang et al., 2015). These methods are all exponential amplification, which causes high false negatives and false positives. In 2017, a new amplification method was invented, which is a linear amplification (Chen et al., 2017, US 10,894,980 referred to as "LIANTI"). False positive rate of SNP detection was much lower than the other methods. However, these kits are not improved for the DNA fragment length characteristics and high protein contained in the culture medium, but only enlarge the reaction system, which results in the amplification efficiency of the culture medium not being high. An efficient and accurate amplification method for obtaining more embryonic DNA information in SCM or/and BF systems is urgently needed. However, the original LIANTI method's yield is too low, and the reproducibility is low, which is not acceptable for clinical application since the embryos are precious.

As for the analysis, there are many reports of non-invasive preimplantation genetic testing for aneuploidy (niPGT-A) (reviewed in Leaver et al., 2020), but only several reports on noninvasive preimplantation genetic testing for monogenic diseases (niPGT-M) (Galluzzi et al., 2015; Zhang et al., 2016; Shangguan et al., 2018; Wu et al., 2015; Liu et al., 2017; Capalbo at al., 2018; Ou et al., 2022). In the reports of niPGT-M by SCM, compared with the trophectoderm biopsy results, the genotype concordance in the successfully amplified SCM also varied greatly, from 21% to 88% (Capalbo et al., 2018; Liu et al., 2017; Ou et al., 2022). The false-positive and false-negative rates were not mentioned in these papers, which are important metrics to assess the accuracy of a testing method. However, based on the genotype concordance rates in the reported articles, the rates of false negatives and false positives would be very high, making noninvasive PGT-M disadvantageous clinically (Capalbo et al., 2018; Cimadomo et al., 2020). Rather than the genotype concordance rate, the most important standard for PGT-M assessment is the misdiagnosis rate. The misdiagnosis rate published by ESHRE PGT consortium was very low (<0.1%) (De Rycke et al., 2017). The risk of misdiagnosis with a new testing method should be assessed and compared with the traditional PGT-M.

In addition to detecting the disease-associated mutation site, the analysis method used in the reported papers was a linkage analysis method. It is recommended that the minimum number of fully informative markers are at least two STRs or SNPs proximal and distal to the mutation site when the Allele Drop-Out (ADO) rates are below 5% (ESHRE 2020). Ou et al. used 4 informative SNPs and Liu et al. used 10 informative SNPs. Since the ADO rates of SCM or BF are much higher than 5%, simply using a few informative sites for linkage analysis can easily lead to misdiagnosis. The distances of the used informative SNP markers to the gene are crucial for the residual recombination risk.

Moreover, maternal contamination further caused diagnostic errors. Widely used linkage analysis methods are based on the Lander-Green algorithm and its variants, which do not consider the maternal contamination and require high quality sequencing data (Lander et al. 1987; Kruglyak et al. 1996; Idury et al. 1997; Kruglyak et al. 1998; Abecasis et al. 2002). Directly applying these methods result in high rates of misdiagnosis (Capalbo et al, 2018; Cimadomo et al. 2020). There are few linkage analysis methods, which has taken into account the mixing or contamination with maternal cells (Fan et al. 2002, Nabieva et al. 2020). However, they still require much higher quality sequencing data than those from culture media, and their main applications are based on blood samples or cells in the placenta or amniotic fluid of the mother during pregnancy. (Fan et al. 2002, Nabieva et al. 2020). These existing methods will cause more diagnostic errors when applied to the sequencing data from culture media. The high false-positive and negative rate of niPGT-M is mainly related to the low initial DNA content in the spent culture medium and the existence of maternal contamination as well as high ADO rate, which make all these existing niPGT-M methods undesirable for clinical application. The key to applying niPGT-M to the clinic IVF is that the accuracy of this technique can reach the level of the current PGT-M. For niPGT-M, the accuracy should be greater than 97% by detecting the existence of the allele that carries the disease's causing mutation in the embryo. To our best knowledge, there hasn't been a linkage-analysis method for a noninvasive procedure. The most critical solution is to develop a more accurate linkage analysis method for samples with high ADO rates in the presence of maternal contamination.

## SUMMARY OF THE INVENTION

The present invention provides a DNA amplification method for amplifying low input amount of DNA in a solution, and a new linkage analysis method based on the Bayesian model to execute PGT-M and preimplantation genetic testing for polygenic diseases (PGT-P) disease-carrying analysis in the presence of maternal contamination and haplotype loss.

Embodiments of the present disclosure provide improved wet lab methods for DNA lysis, whole genome amplification (WGA), and next-generation sequencing (NGS) on limited samples (e.g., clinical spent culture medium, blastocoele fluid, single cells, or a limited number of cells) to achieve high coverage, high accuracy, and high amplification success rate. The system and method described herein are aimed at efficiently amplifying DNA with different fragment distributions in complex systems with high protein content.

This method is modified from the LIANTI method described in the art to result in increased amplification efficiently for higher product yields. LIANTI's sequencing data demonstrated high coverage given the sequencing depth, high accuracy as suggested by the low false positive rate, and low chimera rate. The high accuracy during single-cell genome amplification is a key requisite of single nucleotide variation (SNV) detection, and the low chimera rate is a key requisite of structural variation (SV) detection, both are very important to many applications based on single cell genomics. For example, in some embodiments, the present disclosure provides a method to lyse clinical samples before amplification and a method of amplifying whole-genome samples using a transposon-based method evenly and efficiently, including a) removing serum protein in the clinical samples and changing the lysis temperature and time to expose as much DNA as possible. In some embodiments, the sample of cell-free DNA is in a spent culture medium. In some embodiments, the sample of cell-free DNA is in blastocoele fluid. Aspects of the present disclosure include b) improving subsequent amplification techniques, including but not limited to adjusting protease and/or transposome concentration, adding DNA primers for reverse transcription, increasing amplification cycles in second strand DNA amplification step, etc. Consequently, accurate amplification can be achieved, and the yield can be increased to meet the sequencing analysis. The amplification products can be used in library preparation methods for next-generation sequencing, or for target amplification of disease locus regions, or for chip sequencing.

According to one aspect, the present disclosure provides a linkage analysis method that addresses the problems of high Large Fraction Loss of Haplotype (LFLoH) rate, low coverage, and high Maternal Cell Contamination (MCC) rate that may hinder the inference of inherited parental chromosomes at the disease-causing mutation site. According to the present disclosure, the linkage analysis described herein increases the number of SNPs or markers compared to prior methods to address high ADO rate and further provides a set of linkage analysis methods based on Bayesian analysis, including: a) Calculating the likelihood at each single SNP incorporating the MCC rate and haplotype status of the SNP; (b) Recursively estimating the MCC rate and the haplotype status of each SNP; (c) Calculating the likelihood of observing the SNP data within regions adjacent to the disease-causing mutation site; (d) Determining whether the embryo carries the disease-causing chromosome and providing a level of confidence.

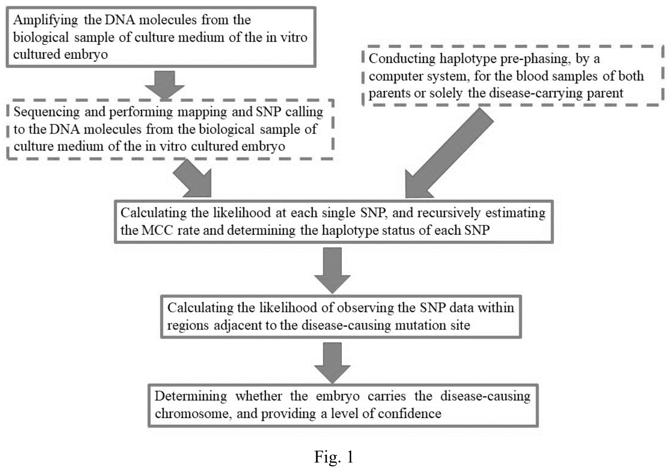

Embodiments of the present disclosure provide an accurate linkage analysis of spent culture medium and/or blastocoele fluid and/or samples having a low input amount of DNA. The present disclosure provides an amplification method to amplify low amounts of DNA in a sample, such as spent culture medium or a single cell. The present disclosure also provides a Bayesian model for assisting PGT-M and PGT-P disease carrying analysis to increase accuracy and lower risk of misdiagnosis, which linkage analysis iteratively calculates the likelihood ratio of inheriting disease-carrying chromosomes versus disease-free chromosomes, incorporating the MCC rate and haplotype status of SNPs and outputs a confidence level for the disease-carrying analysis. The workflow of analysis is shown in Fig. 1. The methods described herein minimize the false negative rate of the output disease-carrying analysis and determine the embryos with the highest possibility of carrying healthy chromosomes. The Bayesian model takes the sequencing data, as well as the parents' haplotypes phased by the Merlin algorithm, as input.

To enhance the data quality, SNPs of extremely low quality are filtered out. Several parameters were estimated before calculating the likelihood ratio: the sequencing error rate, MCC rate and haplotype status. MCC rate was defined as the ratio of DNA fragments sourcing from the maternal chromosome. The haplotype status was defined as the true parental source of the DNA at each SNP and was divided into 'Paternal Chromosome Only', 'Maternal Chromosome Only', or 'Both Parental Chromosome'. Parental chromosome only, for instance, suggested that the DNA detected at this SNP solely came from the father. MCC rate and haplotype status were repeatedly calibrated until convergence.

Then, the likelihood of sequencing data within a specific physical distance to the disease-causing mutation site was recursively calculated. To complete the recursion, the recombination probability between adjacent SNPs is determined and the likelihood at a single SNP is calculated. The recombination probability can be obtained from a long-existing DECODE dataset, while the single-SNP likelihood was attained from a binomial model, whose parameters were determined by the estimated sequencing error rate, MCC rate and haplotype status.

Instead of fixing the number of SNPs considered as in the previously published papers, embodiments of the present disclosure describe adding SNPs one by one from the disease-causing mutation site and calculating the log-likelihood ratio for each SNP subset, which resulted in a curve of the log-likelihood ratio against the physical distance of the end SNP. Typically, the curve started at a point where the ordinate was close to zero and eventually stabilized at a plateau as sufficient SNPs were added. Based on the properties of the curve, which chromosome that had been inherited is determined and the confidence level of our disease-carrying analysis is classified into four categories: Highly Confident, Moderately Confident, Possible and Undetermined. Using this method, alleles can be accurately distinguished in the presence of maternal contamination. This method can make the detection accuracy of niPGT-M reach 100%.

## BRIEF DESCRIPTION OF THE DRAWINGS

Fig. 1 shows the workflow of an embodiment of an exemplary method for an accurate noninvasive PGT.

Fig. 2 shows the mechanism of an exemplary amplification method with added DNA primer.

Fig. 3A-Fig. 3D shows the results of single variable titration of the amplification method, including

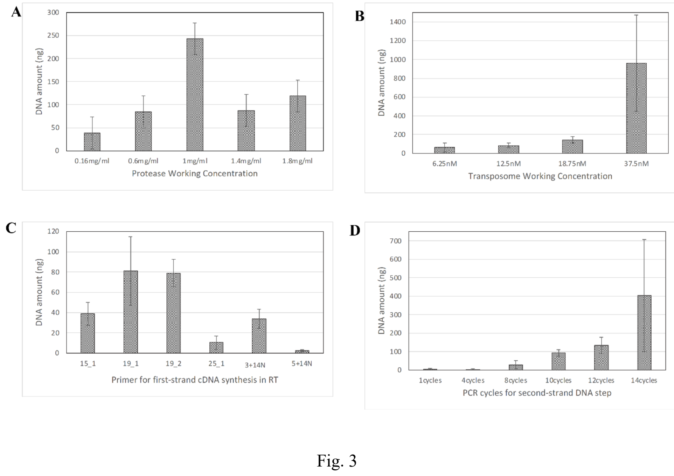

Fig. 3A) protease working concentration, Fig. 3B) transposome working concentration, Fig. 3C) primer for first-strand cDNA synthesis in reverse transcription, and Fig. 3D) PCR cycles for the second-strand DNA step.

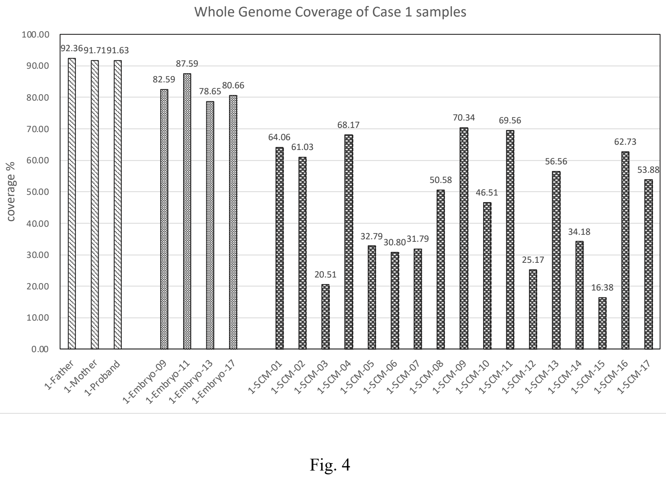

Fig. 4 is a graph showing the genome coverage of each sample in Case 1, including gDNA of patients/proband, amplified discarded embryo DNA, and amplified culture medium DNA.

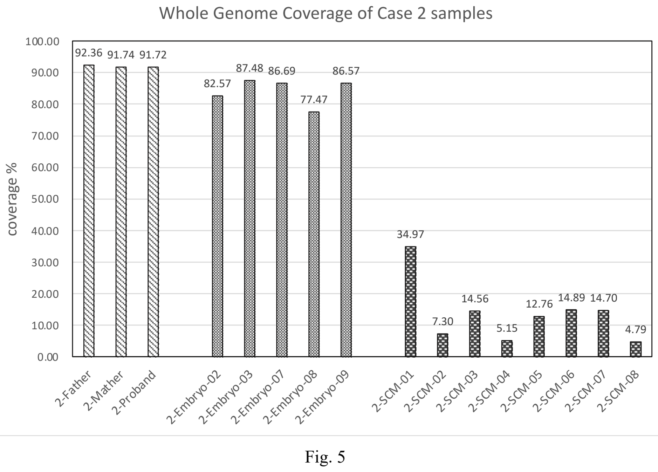

Fig. 5 is a graph showing the genome coverage of each sample in Case 2, including gDNA of patients/proband, amplified discarded embryo DNA, and amplified culture medium DNA.

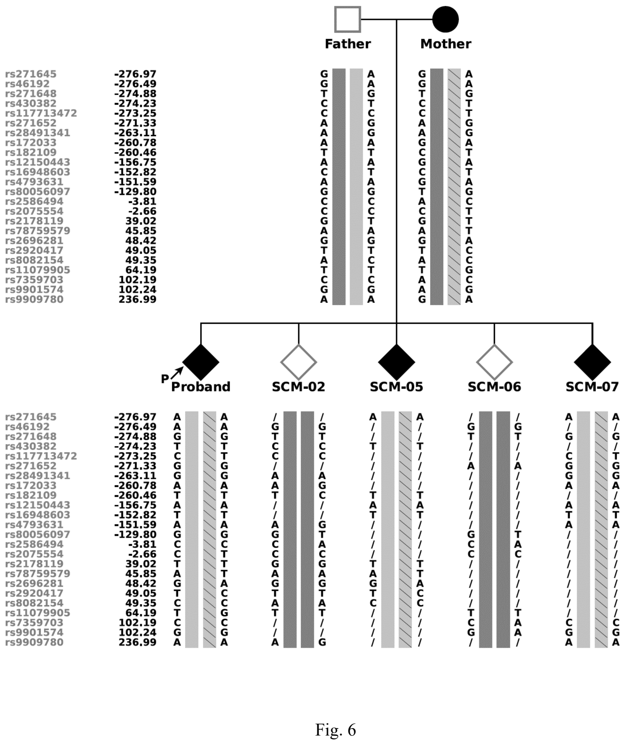

Fig. 6 is a schematic of the Case 1 family's genetic linkage analysis. The chromosome with the slash carries the disease-causing mutation.

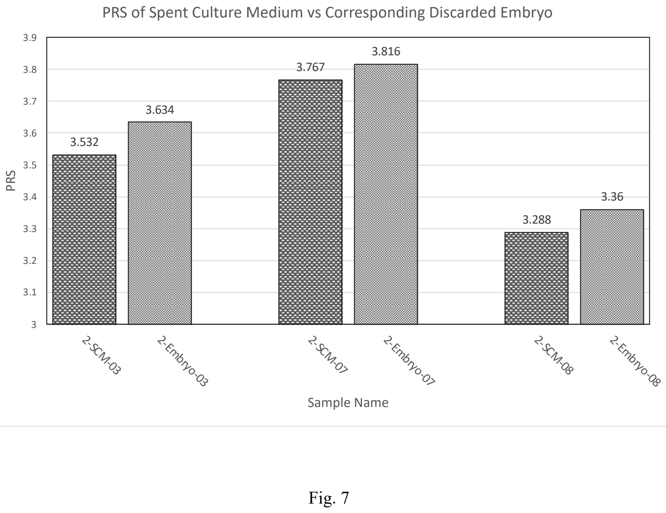

Fig. 7 shows the PRS scores of 3 discarded embryos and their corresponding spent culture media.

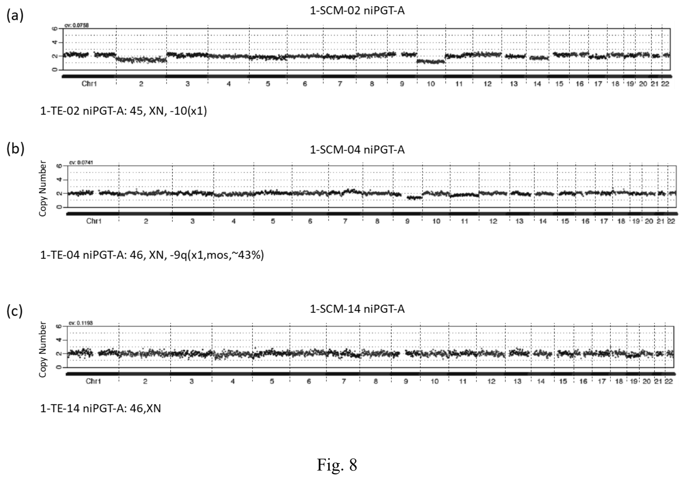

Fig. 8A-Fig. 8C shows the copy number (CN) plots of spent culture medium. Fig. 8A: An aneuploid niPGT-A profile. Fig, 8B: A mosaic niPGT-A profile. Fig. 8C: A euploid niPGT-A profile.

## DETAILED DESCRIPTION OF THE INVENTION

Unless defined otherwise, all technical and scientific terms used herein have the same meaning as is commonly understood by one of skill in the art to which this invention belongs. All patents, patent applications, and publications referred to herein are incorporated by reference.

The term "genetic Disease" as used herein refers to a disorder caused by an abnormality in an individual's DNA or genes. These disorders can be inherited from parents or occur due to spontaneous genetic mutations.

The term "monogenic disease" as used herein refers to a genetic disorder caused by mutations in a single gene. These diseases are typically passed down from one generation to another in a predictable manner, following Mendelian inheritance patterns.

The term "polygenic disease" as used herein refers to a genetic disorder caused by the combined effect of variations in multiple genes. These diseases are often influenced by both genetic and environmental factors and do not follow simple Mendelian inheritance patterns.

The term "solution" as used herein refers to a liquid mixture in which the minor component (e.g., the DNA) is distributed within the major component (e.g., the solvent). Particularly, the solution is a complex system with high protein content, whereas the DNA in the solution can be at the minimum of picogram level. For example, the solution is a spent culture medium or blastocoele fluid.

The term "disease-causing chromosome" as used herein refers to the chromosome or chromosomal region that contains a gene or genes responsible for a particular genetic disease.

The term "disease-causing mutation site" as used herein refers to the specific location within a gene or chromosome where a mutation occurs, leading to the development of a genetic disease.

The term "transposon" as used herein refers to a nucleic acid sequence in DNA that can change its position within a genome. The transposon includes a transposase binding site and an RNA polymerase promoter sequence as described in US 10,894,980 entitled "Methods of Amplifying Nucleic Acid Sequences Mediated by Transposase/Transposon DNA Complexes" directed to a linear amplification via transposon insertion amplification process which may be referred to as "LIANTI" hereby incorporated by reference in its entirety.

The term "transposome" as used herein refers to the set of transposases bound to transposon DNA, which is a transposase/transposon DNA complex dimer.

The term "first strand cDNA synthesis" as used herein refers to forming the single-stranded DNA by reverse transcribing the RNA transcript of the original DNA fragments.

The term "second strand DNA synthesis" as used herein refers to forming a complementary strand to the first strand cDNA after RNA removal by RNase digestion.

The term "allele type" as used herein refers to the specific variant of a gene at a particular location (locus) on a chromosome. Different alleles may have different effects on traits or diseases.

The term "allele depth" as used herein refers to the number of reads (sequences) obtained for a specific allele at a given position. It indicates the abundance of that allele in the sample.

The term "single nucleotide polymorphism (SNP)" as used herein refers to a variation in a single nucleotide base pair within a DNA sequence that occurs among members of a species. SNPs can influence traits, susceptibility to diseases, and other genetic characteristics.

The term "SNP Density" as used herein refers to the number of obtained SNPs in a specific region of the genome from experimental data, divided by the number of all human common SNPs in the database with minor allele frequency larger than 0.01.

The term "haplotype" as used herein refers to a combination of alleles (genetic variants) at multiple loci on the same chromosome that are inherited together due to their physical proximity.

The term "haplotype status" as used herein refers to whether, at a specific SNP location, the allele inherited from the paternal or maternal parent is absent in the experimental data.

The term "haplotype loss" as used herein refers to the situation where certain alleles in a haplotype are lost in the obtained experimental sample.

The term "mapping" as used herein refers to aligning sequence reads to a reference genome to determine their locations.

The term "SNP calling" as used herein refers to the process of identifying SNPs from sequencing data.

The term "phasing" as used herein refers to the process of distinguishing between the paternal and maternal chromosomes within the sequencing sample from any individual or embryo. This differentiation allows the determination of which specific alleles come from each parent. It is important for understanding the inheritance pattern of genetic variants accurately, and understand how alleles are inherited together in haplotypes.

The term "pre-phasing" as used herein refers to phasing the haplotypes of parents before the disease carrying analysis is performed on the biological sample.

The term "recombination probability" as used herein refers to the probability that genetic recombination (exchange of genetic material) will occur between two specific loci on a chromosome during meiosis.

The term "recursive algorithm" as used herein refers to a method that solves a problem by repeatedly applying the same procedure until convergence.

A recitation of "a", "an" or "the" is intended to mean "one or more" unless specifically indicated to the contrary.

## WET LAB

In one aspect, the present invention refers to a method for amplifying the DNA in a solution, wherein the DNA content in the solution can be at the minimum of picogram amounts, the method comprising the following steps:

1) sample lysis: adding lysis buffer into the solution and heating, then adding protease and incubating to fully exposed the DNA, followed by inactivation under high temperatures to obtain the lysate;

2) DNA amplification:

a. transposome having a transposase and a transposon including a transposase binding site and an RNA polymerase promoter sequence is added into the lysate and inserted into the DNA fragment;

b. a 9-bp gap resulting from Tn5 transposon insertion is filled and extended down to both ends of each fragment;

c. performing in vitro transcription using the original sequence as a template;

d. adding DNA primer into a reverse transcription system to form a first strand cDNA via reverse transcription, wherein the DNA primer is complementary to the respective RNA strand of the sequence of the Tn5 transposition binding site; and

e. forming a second strand DNA via cycling amplification.

The transposon-based amplification methods are based on those described in US 10,894,980.

According to one general aspect, a transposition system having an RNA polymerase promoter sequence is combined with an RNA polymerase and reverse transcriptase along with a DNA primer to form a first strand cDNA via reverse transcription, wherein the DNA primer is complementary to the respective RNA strand of the sequence of the Tn5 transposition binding site. According to one aspect, transposons are inserted into DNA within the sample. An RNA polymerase is used to make RNA amplicons which are then reverse transcribed along with the DNA primer into DNA.

Complements to the DNA are made and double stranded genomic DNA sequences are formed. According to one aspect, a method for amplifying double stranded DNA is provided which includes contacting the DNA with transposases (such as TN5 transposases) each bound to a transposon DNA, wherein the transposon DNA includes a double-stranded transposase (Tnp) binding site and an RNA polymerase promoter sequence to form a transposase/transposon DNA complex dimer called a transposome. The transposon DNA may be in the form of a single stranded extension or in the form of a loop with each end connected to a corresponding strand of the double stranded transposase binding site. The transposome bind to DNA target locations along the double stranded DNA and cleave the double stranded DNA into a plurality of double stranded fragments, with each double stranded fragment having a first complex attached to an upper strand by the Tnp binding site and a second complex attached to a lower strand by the Tnp binding site. The transposon binding site is attached to each 5' end of the double stranded fragment. According to one aspect, the transposases are removed from the complex. The double stranded fragments are extended along the transposon DNA to make a double stranded extension product having T7 promoters at each end. According to one aspect, a gap which may result from attachment of the transposase binding site to the double stranded genomic DNA fragment may be filled and extended. The double stranded extension product is contacted with RNA polymerase (such as T7 RNA polymerase) to make an RNA transcript of the double stranded extension product. According to one aspect, a plurality of RNA transcripts of the double stranded extension product are made using RNA polymerase. The RNA transcripts are reverse transcribed into a plurality of corresponding single stranded DNA using a reverse transcriptase and a DNA primer complementary to the respective RNA strand of the sequence of the Tn5 transposition binding site. Complementary strands to the single stranded DNA are created to form a plurality of double stranded DNA. The double stranded DNA may then be amplified and sequenced using, for example, high-throughput sequencing methods known to those of skill in the art.

According to certain aspects, an exemplary transposon system is a Tn5 transposon system. Other useful transposon systems are known to those of skill in the art and include Tn3 transposon system (see Maekawa, T., Yanagihara, K., and Ohtsubo, E. (1996), A cell-free system of Tn3 transposition and transposition immunity, Genes Cells 1, 1007-1016), Tn7 transposon system (see Craig, N.L. (1991), Tn7: a target site-specific transposon, Mol. Microbiol. 5, 2569-2573), Tn10 tranposon system (see Chalmers, R., Sewitz, S., Lipkow, K., and Crellin, P. (2000), Complete nucleotide sequence of Tn10, J. Bacteriol 182, 2970-2972), Piggybac transposon system (see Li, X., Burnight, E.R., Cooney, A.L., Malani, N., Brady, T., Sander, J.D., Staber, J., Wheelan, S.J., Joung, J.K., McCray, P.B., Jr., et al. (2013), PiggyBac transposase tools for genome engineering, Proc. Natl. Acad. Sci. USA 110, E2279-2287), Sleeping beauty transposon system (see Ivics, Z., Hackett, P.B., Plasterk, R.H., and Izsvak, Z. (1997), Molecular reconstruction of Sleeping Beauty, a Tc1-like transposon from fish, and its transposition in human cells, Cell 91, 501-510), Tol2 transposon system (see Kawakami, K. (2007), Tol2: a versatile gene transfer vector in vertebrates, Genome Biol. 8 Suppl. 1, S7.)

According to certain aspects, an exemplary RNA polymerase is T7 RNA polymerase. Other useful RNA polymerases are known to those of skill in the art and include T3 RNA polymerase (see Jorgensen, E.D., Durbin, R.K., Risman, S.S., and McAllister, W.T. (1991) Specific contacts between the bacteriophage T3, T7, and SP6 RNA polymerases and their promoters, J. Biol. Chem. 266, 645-651), and SP6 RNA polymerase (see Melton, D.A., Krieg, P.A., Rebagliati, M.R., Maniatis, T., Zinn, K., and Green, M.R. (1984) Efficient in vitro synthesis of biologically active RNA and RNA hybridization probes from plasmids containing a bacteriophage SP6 promoter, Nucleic Acids Res. 12, 7035-7056.)

According to one aspect, transposon DNA is designed to contain a double-stranded 19 bp Tn5 transposase (Tnp) binding site at one end, linked or connected, to a strong T7 promoter sequence. Upon transposition, the Tnp and the transposon DNA bind to each other and dimerize to form transposomes. The transposomes then randomly capture or otherwise bind to DNA in the sample. Then, the transposases in the transposome cut the DNA with one transposase cutting an upper strand and one transposase cutting a lower strand to create a DNA fragment. The transposon DNA is thus inserted randomly into the DNA, leaving a 9-bp gap on both ends of the transposition/insertion site. The result is a DNA fragment with a transposon DNA Tnp binding site attached to the 5' position of an upper strand and a transposon DNA Tnp binding site attached to the 5' position of a lower strand.

After transposition, gap extension is performed to fill the 9 bp gap and complement the T7 promoter sequence originally designed in the transposon DNA. As a result, active double-stranded T7 promoter sequences are attached to both ends of each DNA fragment. Next, T7 RNA polymerase is added, and in vitro transcription is used to linearly amplify the DNA fragments, generating many RNAs that contain the same sequence as the original double stranded DNA template. Finally, by reverse transcription using a reverse transcriptase and a DNA primer as described above to form DNA followed by second strand synthesis, the amplified RNAs are converted back into double-stranded DNA molecules. The DNA fragments may then be further processed for standard library preparation, amplification and sequencing.

Particular Tn5 transposition systems are described and are available to those of skill in the art. See Goryshin, I.Y. and W.S. Reznikoff, Tn5 in vitro transposition. The Journal of biological chemistry, 1998. 273(13): p. 7367-74; Davies, D.R., et al., Three-dimensional structure of the Tn5 synaptic complex transposition intermediate. Science, 2000. 289(5476): p. 77-85; Goryshin, I.Y., et al., Insertional transposon mutagenesis by electroporation of released Tn5 transposition complexes. Nature biotechnology, 2000. 18(1): p. 97-100 and Steiniger-White, M., I. Rayment, and W.S. Reznikoff, Structure/function insights into Tn5 transposition. Current opinion in structural biology, 2004. 14(1): p. 50-7 each of which are hereby incorporated by reference in their entireties for all purposes. Kits utilizing a Tn5 transposition system for DNA library preparation and other uses are known. See Adey, A., et al., Rapid, low-input, low-bias construction of shotgun fragment libraries by high-density in vitro transposition. Genome biology, 2010. 11(12): p. R119; Marine, R., et al., Evaluation of a transposase protocol for rapid generation of shotgun high-throughput sequencing libraries from nanogram quantities of DNA. Applied and environmental microbiology, 2011. 77(22): p. 8071-9; Parkinson, N.J., et al., Preparation of high-quality next-generation sequencing libraries from picogram quantities of target DNA. Genome research, 2012. 22(1): p. 125-33; Adey, A. and J. Shendure, Ultra-low-input, tagmentation-based whole-genome bisulfite sequencing. Genome research, 2012. 22(6): p. 1139-43; Picelli, S., et al., Full-length RNA-seq from single cells using Smart-seq2. Nature protocols, 2014. 9(1): p. 171-81 and Buenrostro, J.D., et al., Transposition of native chromatin for fast and sensitive epigenomic profiling of open chromatin, DNA-binding proteins and nucleosome position. Nature methods, 2013, each of which are hereby incorporated by reference in their entireties for all purposes. See also WO 98/10077, EP 2527438 and EP 2376517 each of which are hereby incorporated by reference in their entireties. A commercially available transposition kit is marketed under the name NEXTERA and is available from Illumina.

In vitro transcription (IVT) by T7 RNA polymerase is useful to create RNA amplicons. See Van Gelder, R.N., et al., Amplified RNA synthesized from limited quantities of heterogeneous cDNA. Proceedings of the National Academy of Sciences of the United States of America, 1990. 87(5): p. 1663-7; Kawasaki, E.S., Microarrays and the gene expression profile of a single cell. Annals of the New York Academy of Sciences, 2004. 1020: p. 92-100; Livesey, F.J., Strategies for microarray analysis of limiting amounts of RNA. Briefings in functional genomics & proteomics, 2003. 2(1): p. 31-6; Tang, F., K. Lao, and M.A. Surani, Development and applications of single-cell transcriptome analysis. Nature methods, 2011. 8(4 Suppl): p. S6-11; Hashimshony, T., et al., CEL-Seq: single-cell RNA-Seq by multiplexed linear amplification. Cell reports, 2012. 2(3): p. 666-73; and Shankaranarayanan, P., et al., Single-tube linear DNA amplification for genome-wide studies using a few thousand cells. Nature protocols, 2012. 7(2): p. 328-38 each of which are hereby incorporated by reference in their entireties. According to the present disclosure, IVT advantageously provides linear amplification, with all the copies generated from the original DNA template. The resulting RNA molecules can be reverse transcribed into single stranded DNA followed by complementary strand formation resulting in double stranded DNA, which are amplicons linearly amplified from the original DNA template.

In one embodiment, the solution is a culture medium or a blastocoele fluid. Particularly, the culture medium or the blastocoele fluid has high protein content and low input amount of DNA. According to one aspect described below, the method is applied to culture medium as merely exemplary.

In one embodiment, the lysis buffer is 2x to 10x lysis buffer. The original volume of the culture medium is much larger than that for a single cell. The amplification of as much proportion of the culture medium as possible was achieved by using a very small amount of highly concentrated lysis buffer. This results in the largest percentage amount of culture medium DNA to be used as a template in this amplification system.

In one embodiment, the working concentration of the protease in the lysis mixture is higher than 0.6 mg/mL, preferably in the range of 0.8-2 mg/mL. For example, the working concentration of the protease is 0.6 mg/mL, 0.8 mg/mL, 1 mg/mL, 1.2 mg/mL, 1.4 mg/mL, 1.6 mg/mL, 1.8 mg/mL, 2 mg/mL or higher. The amount of protease is increased to remove the extra exogenous serum proteins. In one embodiment, the incubation is performed at 45-60°C for 1 to 3 hours.

In one embodiment, the inactivation is performed at 80-90°C. The protease reaction time is shortened and the protease inaction temperature is increased to ensure that there is no excess protease residue in the subsequent reaction.

In one embodiment, the working concentration of the transposome is higher than 6 nM, preferably at the range of 10-40 nM. For example, the working concentration of the transposome is 6 nM, 6.25nM, 8 nM, 10 nM, 12.5 nM, 14 nM, 16 nM, 18.75 nM, 20 nM, 22 nM, 24 nM, 26 nM, 28 nM, 30 nM, 32 nM, 34 nM, 36 nM, 37.5 nM, 40 nM or higher.

In one embodiment, the DNA primer has a length of 15bp to 25bp. Preferably, the 3'-end of the DNA primer contains the sequence of 5'-GACAG-3' or 5'-CTGTC-3'. For example, the random primer 5'-NNNNNNNNNNNNNNNGA-CAG-3' or 5'-NNNNNNNNNNNNNNNCTGTC-3' can be used. More preferably, the DNA primer contains the sequence set forth as 5'-AGATGTGTATAAGAGACAG-3' (Seq. ID No.: 1), or 5'-TCTACACATATTCTCTGTC-3' (Seq. ID No.: 2), or has at least 70%, preferably 80%, preferably 90%, preferably 95, preferably 100% identity with Seq. ID No.: 1 or Seq. ID No.: 2. For example, primer 5'-AGATGTGTATAAGAG-3' can be used.

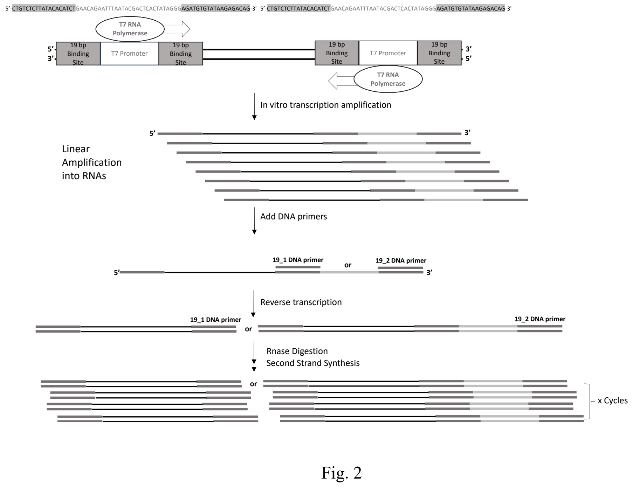

The distribution of the DNA in the culture medium was measured and it was determined that both long and short fragments of cell-free DNA were present in the culture medium. Since current commercialized kits all tend to amplify the long fragments, transposon-based amplification methods described herein based on those described in US 10,894,980 as modified herein are designed to amplify both long (e.g., greater than 1kb, in some embodiments) and short fragments (e.g., 100bp~600bp, or less than 1kb, in some embodiments). The transposon amplification methods described herein do not use self-priming and use a DNA primer added with proper concentration during the reverse transcription step for the highest efficiency. The RNAs generated in *in vitro* transcription are specialized by a sequence of complete transposon DNA sequence at the 3' end. Two primers designed for the reverse transcription, named 19_1 and 19_2, and their binding positions are shown in Fig. 2.

In one embodiment, the cycling amplification is performed more than 4 cycles, such as from 4 to 20 cycles, preferably 6 to 15 cycles. After the formation of the first strand of cDNA in reverse transcription, additional amplification cycles were used to amplify the second strand of DNA. Linear amplification, such as described in US 10,894,980 and C Chen, D Xing, L Tan, H Li, G Zhou, L Huang, XS Xie, Science 2017; 356 (6334): 189-194, is a high-precision amplification, however, the product yield of linear amplification is lower than exponential amplification. In order to facilitate clinical testing methods, the present disclosure provides methods that increase the yield of amplification. In one embodiment, this is accomplished during the reaction where the second strand is formed by increasing the number of the amplification cycles in order to obtain enough DNA for sequencing library preparation.

The methods disclosed herein achieve an amplification success rate of nearly 100% while keeping the fidelity for amplification.

## DRY LAB

In another aspect, the present invention refers to a method of analyzing a biological sample of culture medium of an in vitro cultured embryo to determine the susceptibility of a genetic disease in the embryo, comprising:

1) Amplifying the DNA molecules from the biological sample of culture medium of the in vitro cultured embryo and/or, for reference only, the biological sample of a discarded embryo in the family;

2) Preparing DNA libraries and sequencing the DNA molecules from the biological sample to obtain a plurality of sequence reads;

3) Performing mapping and SNP calling, such as by a computer system, to generate the SNP data and controlling its quality;

4) Conducting haplotype pre-phasing, such as by a computer system, for the blood samples of both parents or solely the disease-carrying parent;

5) Calculating, such as by a computer system, the likelihood of the four possible inheritance scenarios at each single SNP representing whether the genetic chains are inherited from the paternal or maternal alleles of the parents, taking into account the maternal cell contamination (MCC) rate and haplotype status;

6) Estimating the MCC rate, such as by a computer system, and determining the haplotype status of each SNP to represent whether only DNA from one parent is present in the culture medium;

7) Calculating, such as by using a computer system, the likelihood of observing the SNP data within regions adjacent to the disease-causing mutation site, combining the likelihood at each single SNP and the recombination probabilities;

8) Determining whether the embryo carries the disease-causing chromosome, and providing a level of confidence.

In some embodiments disclosed herein, aforementioned step 5 and step 6 provide specific, technological improvements over some existing techniques that use SNP data to determine whether an embryo carries a disease-causing chromosome. For example, if the wet lab provides very high quality biological samples that result in highly precise and accurate SNP data without maternal contamination from the samples, then one or more computations performed in step 5 and step 6 might offer only marginal improvement in accuracy. For example, methods for amplifying (as recited in aforementioned step 1) minute amounts of DNA in a solution are sometimes used to improve the quality of resulting SNP data. Nevertheless, despite attempts at improving the quality (e.g., staying free of contaminants, amplifying, etc.) of biological samples, this is not always possible to the desired extent or level of confidence. Moreover, high ADO rates and maternal contamination are the intrinsic property of the DNA content in certain biological samples, such as a biological sample of culture medium from an in vitro cultured embryo, and are inevitable even if the wet lab can reach near-perfect performance. In contrast to prior art systems, the novel and nonobvious computations performed in step 5 and step 6 provide an improvement in the precision with which a biological sample of culture medium from an in vitro cultured embryo is analyzed to more accurately determine the susceptibility of a genetic disease in the embryo. Rather than being solely dependent on the wet lab and its known techniques to provide just very high quality biological samples for linkage analysis and/or non-invasive preimplantation genetic testing, this disclosure includes one or more additional computational operations performed in step 5 and/or step 6 to overcome various shortcomings in the prior art. In other words, prior art systems do not have a linkage-analysis method for a non-invasive procedure similar to the features disclosed herein-for example, several embodiments disclosed herein enable a markedly accurate linkage analysis method for even those samples with high ADO rates in the presence of maternal contamination. Steps 1 to 8 are further elaborated as follows.

Step 1): Amplifying the DNA molecules from the biological sample of culture medium of the in vitro cultured embryo and/or, for reference only, the biological sample of a discarded embryo in the family.

In one embodiment, the embryo has contamination caused by maternal cells and/or haplotype loss. The biological sample of a discarded embryo in the family is used for the following deduction of pre-phasing when neither a proband nor grandparents are present, which will be discussed in detail below.

Step 2): Preparing DNA libraries and sequencing the DNA molecules from the biological sample to obtain a plurality of sequence reads.

The amplicons were sheared and library preparation was performed. The DNA sequencing libraries were sequenced on an Illumina platform to generate raw sequencing data.

Step 3): Performing mapping and SNP calling, by a computer system, to generate the SNP data and controlling its quality.

Raw paired-end reads were trimmed Illumina sequencing adapters, inline barcodes, binding sequences and T7 promoter sequences, and low-quality ends by Cutadapt. Clean reads were mapped to human reference genome hg19 with BWA mem. Each BAM file was marked duplicates and sorted by MarkDuplicatesSpark of GATK 4.2.0. Base quality score recalibration (BQSR) was performed using BaseRecalibrator to generate a recalibration table, followed by ApplyBQSR to adjust the base quality accordingly. For variant calling, GATK HaplotypeCaller produced a genomic variant call format (GVCF) file and VCF file for each sample. GenotypeGVCFs was utilized to perform joint genotyping across all samples. SNPs were filtered using variant quality score recalibration (VQSR) with HapMap 3.3, Omni 2.5, 1000 Genome phase I, and dbSNP 151 as SNP training sets. A 99% sensitivity threshold was chosen to filter SNPs accurately. Biallelic SNPs were retained for subsequent analysis. The VCF contains the chromosome number, the physical location, identification number (ID), and allele type as well as the sequencing quality, sequencing depth (DP, Depth), and allele depth (AD) for each SNP of each blood sample, the biological sample of the culture medium of the in vitro cultured embryo and/or the biological sample of the discarded embryo in the family.

SNPs with two different alleles are considered and all SNPs with DP values less than 5, or sequencing quality values (QUAL) less than 30, or gene quality (GQ) values less than 10 in the blood samples are excluded, because they are considered to be of too poor quality and too small signal-to-noise ratio.

After removing the low-quality SNPs, 10MB (Million Base pairs) upstream and downstream of the disease-causing mutation site in the biological sample is defined as the Adjacent Region of the disease-causing mutation site. The Sequencing Error Rate (SER) in the Adjacent Region is estimated. SNPs with genotype 00 of both father and mother, or genotype 11 of both father and mother are selected. Ideally, at such SNPs, genotypes that are identical to the parents' genotype should only be detected. Therefore, sequencing data with genotypes different from those of the parents must be the wrong reads. Hence, the estimated Sequencing Error Rate (SER) is defined as the proportion of the total number of erroneous reads versus the total number of reads.

The relative density of SNPs detected in the adjacent region of the disease-causing mutation site is estimated, and defined as the ratio of the total number of SNPs detected in this area compared to the total number of human common SNPs within this region. Here, human common SNPs are defined as all SNPs with Minor Allele Frequency (MAF) greater than or equal to 0.01, and the Minor Allele Frequency of a group of alleles refers to the frequency of its second most frequent allele in a population. The human common SNP data updated in 2018 by dbSNP [Sherry et al., 2001] is used to calculate the relative SNP density for each sample.

After completing the above steps, the sample of culture medium with an abnormal MCC rate (>0.65 or <-0.5) or low SNP density (<0.001) or a high sequencing error rate (>0.2) is considered to be of extremely low quality and thus excluded. Such culture medium are categorized into "Undetermined" and the reasons as low data quality are outputted. All the samples passing the quality control will come into the next stage.

Step 4): Conducting haplotype pre-phasing, by a computer system, for the blood samples of both parents or solely the disease-carrying parent.

To deduce parental haplotypes, genomic data of any one or more of the following samples in the family is used: the blood sample of proband, or the blood sample of at least one of the grandparents. When neither a proband nor grandparents are present, discarded embryos are used, for reference only, for the deduction. Since discarded embryos, for reference only, are the samples after amplification, which will have errors in the process of amplification to be introduced, in order to ensure the accuracy of the deduction, a minimum of two discarded embryo data are used in parallel to perform the deduction and correct each other for Mendelian errors.

Plink 1.9 with the option "--mendel" is used to identify sites with Mendelian errors. Additionally, BEAGLE 4.0 is used to phase the genotype data, taking into account the parents-offspring relationships and simultaneously eliminating sites that contain Mendelian errors.

Step 5): Calculating the likelihood of the four possible inheritance scenarios at each single SNP representing whether the genetic chains are inherited from the paternal or maternal alleles of the parents, taking into account the maternal cell contamination (MCC) rate and haplotype status.

For the single-SNP likelihood, the binomial distribution is used to build a model (see the appendix section of Nabieva et al. [2020]), where the parameters of the binomial distribution used were the sequencing error rate $\widehat{SER}$, MCC rate $\hat{\alpha}$, and detected AD values of the two alleles at each single SNP. Specifically, the following

$$l_{11} = \left(\frac{1-\hat{\alpha}}{2}, 0, \frac{1}{2}, \frac{\hat{\alpha}}{2}\right)^{\top}, \quad l_{12} = \left(\frac{1-\hat{\alpha}}{2}, 0, \frac{\hat{\alpha}}{2}, \frac{1}{2}\right)^{\top},$$

$$l_{21} = \left(0, \frac{1-\hat{\alpha}}{2}, \frac{1}{2}, \frac{\hat{\alpha}}{2}\right)^{\top}, \quad l_{22} = \left(0, \frac{1-\hat{\alpha}}{2}, \frac{\hat{\alpha}}{2}, \frac{1}{2}\right)^{\top}.$$

defines as the theoretical proportion of four chromosomes (paternal inherited chromosome, paternal non-inherited chromosome, maternal inherited chromosome, maternal non-inherited chromosome) in embryonic culture medium. To take LFLoH (See below for definition) into consideration, the two formulae below

$$h(\text{H}) = \begin{cases} (1,1,0,0)^{\top} & \text{If H} = \text{F} \\ (0,0,1,1)^{\top} & \text{If H} = \text{M} \\ (1,1,1,1)^{\top} & \text{If H} = \text{FM or } H = \text{Unknown} \\ (1,0,0,0)^{\top} & \text{If H} = \text{F}_1 \\ (0,1,0,0)^{\top} & \text{If H} = \text{F}_2 \end{cases}$$

and

$$\widehat{l_{ij}} = \frac{l_{ij} \circ h\left(H_n\right)}{l_{ij}^\top h\left(H_n\right)}.$$

are used, where H is the status of haplotype at this SNP. Here, ∘ denotes product by position.

Paternal genotype at the n-th SNP is denoted as fGT = fGT1/fGT2 and the maternal one as mGT = mGT1/mGT2, where fGT1, fGT2, mGT1, mGT2 $\in$ {0, 1}. The following

$$\mathbf{GT_n} = (\mathbf{fGT_1}, \mathbf{fGT_2}, \mathbf{mGT_1}, \mathbf{mGT_2})^\top,$$

is denoted as a four-dimensional vector and denotes

$$\hat{p}_{ij}^{(n)} = \hat{l}_{ij}^{\mathbf{T}} \cdot \mathbf{GT_n},$$

then $\hat{p}_{ij}^{(n)}$ is the theoretical probability that an ALT gene is obtained by randomly sampling one read at n-th SNP in the culture medium when MCC rate and haplotype loss are taken into consideration.

Therefore, the single-SNP likelihood is formulated as

$$\hat{L}_{ij}^{(n)} = \frac{\mathrm{AD_n}!}{\mathrm{AD_{0,n}!} \cdot \mathrm{AD_{1,n}!}} \cdot \left[\hat{p}_{ij}^{(n)} \widehat{SER} + \left(1 - \hat{p}_{ij}^{(n)}\right)\left(1 - \widehat{SER}\right)\right]^{\mathrm{AD_{0,n}}}$$

$$\cdot \left[\hat{p}_{ij}^{(n)}\left(1 - \widehat{SER}\right) + \left(1 - \hat{p}_{ij}^{(n)}\right)\widehat{SER}\right]^{\mathrm{AD_{1,n}}} \quad \text{for } i, j \in \{1, 2\}$$

In some embodiments, the same computer system may perform all of the steps of the method for determining the susceptibility of a genetic disease in the embryo. In other embodiments, one or more distinct computer processors in a computer system (e.g., computer subsystems) may perform one or more of the steps of the afore-mentioned method without necessarily performing all of them. In other words, the computer system may comprise multiple subsystems, each with its own computer processor, so that the computational load of performing the numerous steps recited in the afore-mentioned method is distributed.

Step 6): Estimating the MCC rate, and determining the haplotype status of each SNP to represent whether only DNA from one parent is present in the culture medium.

Step 6) further includes the following steps:

Step (a): Initially estimating the MCC rate and haplotype status of each SNP in the region adjacent to the disease-causing mutation site.

Maternal Cell Contamination rate (MCC rate) is defined as the proportion of maternal DNA in the culture medium of an embryo compared to the total amount of DNA and this value should theoretically be between 0 and 1. All SNPs with paternal genotype 00 and maternal genotype 11, or with paternal genotype 11 and maternal genotype 00 in the adjacent region are selected. At these SNPs, it is determined theoretically whether each read detected in the culture medium is from the father or the mother, and the proportion of reads from the mother over all reads is calculated and used as an initial estimate of MCC rate. In contrast to prior art systems which omit a linkage-analysis method for a non-invasive procedure similar to the features disclosed herein, this disclosure provides for various computational operations on SNP data that enable a more accurate linkage analysis method even for samples with high ADO rates in the presence of maternal contamination, as explained herein. In particular, in wet labs using non-invasive procedures, poor data quality can sometimes occur because missing DNA fragments from one parent are often longer in length and contain dozens or more SNPs, thus the ability to sequence is negatively effected and diminished. The computational operations disclosed herein solve a real world, technological problem by enabling an otherwise unusable biological sample to become useful to determine the susceptibility of a genetic disease in an embryo.

In some examples, a method of preparation is disclosed herein to prepare an otherwise unsequenceable biological sample of culture medium of an in vitro cultured embryo where the underlying biological sample is low quality for, among other things, having high ADO rates in the presence of maternal contamination. The preparation method transforms the SNP data resulting from the otherwise unsequenceable biological sample into a state that is capable of being analyzed to determine a susceptibility of a genetic disease in the embryo. For purposes of determining the susceptibility of a genetic disease in an embryo, low quality DNA samples result in mostly unsequencable data. Moreover, DNA in embryos enter the culture medium in fragments, so if certain longer DNA fragments do not enter the culture medium, it results in the inability of sequencing (e.g., a phenomenon defined as Large Fragment Loss of Haplotypes (LFLoH) herein). The present disclosure describes a method of preparation of the seemingly unsequencable data into a transformed state where the resulting SNP data overcomes the shortcomings of LFLoH, and the previously unusable biological sample is now rendered usable. Because the resulting SNP data from the wet lab is based on a biological sample with less than ideal quality due to aforementioned circumstances, novel and nonobvious computational steps are performed on the SNP data to transform the seemingly unusable data into an usable one; in some embodiments, particularly for the purposes of determing whether an embryo awaiting implantation carries the disease-carrying chromosome or not. Because of the innovative computational steps disclosed herein, another round of sequencing in the wet lab may be avoided, thus saving on time, supplies, labor, and other resources. As elaborated below in step 6(b), analyzing LFLoH provides insights into whether parental DNA fragments are detected at each SNP, which may result in the final haplotype status labels at all SNPs to be iteratively updated to be more accurate until the MCC rate converges or other final estimation criterion is reached.

Notably, the afore-mentioned method of preparation is different from a method of treatment. The method of preparation recited herein is more than simply identifying a natural correlation between two naturally occurring properties of biological samples. For example, simply using the natural correlation between heightened cfDNA levels in a collected bodily sample and organ transplant health, to identify a potential organ rejection might not be sufficient to recite patent eligible subject matter. In contrast, in some examples disclosed herein, the computational method disclosed in step 5 and/or step 6 enriches a previously unusable biological sample into a transformed state that provides to-be-parents with an accurate, non-invasive verification of the susceptibility of a genetic disease in their embryo. By avoiding invasive procedures such as sticking a big needle into the amniotic sac of a pregnant woman, a number of deaths of to-be-mothers and fetuses can be avoided. The disclosed method of preparation provides a technical solution to a real world problem that previously resulted in higher risk to fetuses, women, and their health.

Ideally, it is advantageous to be able to detect DNAs from both parents in the embryo culture medium. However, in practice, in embryo culture medium, it has been observed that a significant portion of the genome can only be detected from one parent. Such a type of DNA loss is different from traditional sense of Allele Drop-Out (ADO), which arises due to amplification heterogeneity, resulting in some short DNA fragments that are not successfully amplified in a certain round of amplification, and thus are amplified significantly less than other DNA fragments to be detected. It reflects the efficiency of amplification methods, and it involves DNA fragments that are usually short and contain only about 1 or 2 SNPs. However, in the culture medium samples obtained by non-invasive methods, the missing DNA fragments from one parent are often longer in length and contain dozens or more SNPs. Such haplotype loss is not due to low amplification efficiency, but rather due to poor data quality. DNA in embryos enters the culture medium in fragments, so if certain longer DNA fragments do not enter the culture medium, it results in the inability of sequencing. We define Large Fragment Loss of Haplotypes (LFLoH) as the phenomenon that long and contiguous DNA fragments from one or both parents do not enter the culture medium and thus cannot be detected in sequencing. The present disclosure provides a model that addresses LFLoH in a two-step process.

First, SNPs that can be directly determined from their AD values are labeled as either containing only paternal DNA (F), only maternal DNA (M), or both (FM) Others remain unlabeled.

In the second step, the surrounding SNPs in the upstream and downstream of these labeled SNPs are searched, and it is then determined whether the surrounding SNPs could be labeled with the same label as the labeled SNP in the first step. Specifically, in the first step, T is assumed to be an integer threshold determined in advance (the default value is 3).

For SNP in the adjacent region of the disease-causing mutation site, those satisfying one of the following conditions are labeled as F.

- The paternal genotype is 00 and the maternal genotype is 11. $AD_0 > T$ and $AD_1 = 0$.
- The paternal genotype is 11 and the maternal genotype is 00. $AD_1 > T$ and $AD_0 = 0$.
- The paternal genotype is 01 and the maternal genotype is 00. $AD_0 = 0$ and $AD_1 > T$.
- The paternal genotype is 01 and the maternal genotype is 11. $AD_0 > T$ and $AD_1 = 0$.

Some SNPs labeled as F can be further labeled as F1 or F2, indicating that the detected SNP is from the paternal source chromosome or the maternal source chromosome of the father. Specifically, SNP labeled F is labeled as F1, if the SNP satisfies one of the following.

- The paternal genotype is 1|0 and the maternal genotype is 00. $AD_1 > T$ and $AD_0 = 0$.

- The paternal genotype is 0|1 and the maternal genotype is 11. $AD_0$ > T and $AD_1$ = 0,

where 0|1 and 1|0 denote the paternal genotype, and the number at left hand side and right hand side of | denote the paternal source and maternal source gene of the father at this SNP respectively. Similarly, a SNP labeled F is labeled as F2, if the SNP satisfies one of the following two conditions.

- The paternal genotype is 0|1 and the maternal genotype is 00. $AD_1$ > T and $AD_0$ = 0.
- The paternal genotype is 1|0 and the maternal genotype is 11. $AD_0$ > T and $AD_1$ = 0,

SNPs satisfying one of the following conditions are labeled as M.

- The paternal genotype is 00 and the maternal genotype is 11. $AD_1$ > T and $AD_0$ = 0.
- The paternal genotype is 11 and the maternal genotype is 00. $AD_0$ > T and $AD_1$ = 0.

Due to the existence of MCC, no SNP is labeled as M1 or M2. SNPs satisfying one of the following conditions are labeled as FM.

- The paternal genotype is 00 and the maternal genotype is 11. $AD_1$ > [T/2] and $AD_0$ > [T/2];
- The paternal genotype is 00 and the maternal genotype is 11. $AD_1$ > [T/2] and $AD_0$ > [T/2];
- The paternal genotype is 01 and the maternal genotype is 11. $AD_1$ > [T/2] and $AD_0$ > [T/2];
- The paternal genotype is 01 and the maternal genotype is 00. $AD_1$ > [T/2] and $AD_0$ > [T/2]; where [] denotes the floor function.

After completing the initial labeling procedure, there are 5 possible labels: F, M, FM, F1, F2. Suppose there are several labeled SNPs among the adjacent region of the disease-causing mutation site, with an ordinal number $-N_1 \leq s_1 < s_2 < ... < s_D \leq N_2$. Then, for a particular SNP $s_d$ that has been labeled, a search is carried out between the SNPs with ordinal number $s_d$-1 and $s_d$+1. The $s_d$-th SNP is called the central SNP and the region between $s_d$-1-th and $s_d$+1-th SNP is called the searching region. The search region is divided into two parts: the upstream part and the downstream part of the $s_d$-th SNP. In the downstream searching, each SNP in the downstream searching region is assigned a label. The n-th SNP during downstream searching from its nearest labeled SNP upstream from it is denoted as $H_n^{down}$ and during upstream searching from its nearest labeled SNP downstream from it is denoted as $H_n^{up}$.

The search process only in the downstream region is described as follows as an example. First, $H_{s_d}^{down}$ is initialized as the label assigned in the first labeling step. Next, assume $s_d, s_d$ +1, ..., $s_d$ +m-1 are all labeled the same as $H_{s_d}^{down}$. The next step is to search the ($s_d$ + m)-th SNP and determine whether the same label can be assigned to this SNP. The same label is assigned to ($s_d$ + m)-th SNP if and only if

$$\frac{\sum_{i,j=1}^2 \mathbb{P}\left(\mathrm{AD}_{s_d:s_d+m}\middle|\mathrm{F}_{s_d} = i, \mathrm{M}_{s_d} = j, H_{s_d:s_d+m}^{\mathrm{down}} = H_{s_d}^{\mathrm{down}}\right)}{\sum_H \sum_{i,j=1}^2 \mathbb{P}\left(\mathrm{AD}_{s_d:s_d+m}\middle|\mathrm{F}_{s_d} = i, \mathrm{M}_{s_d} = j, H_{s_d:s:d+m-1}^{\mathrm{down}} = H_{s_d+m}^{\mathrm{down}} = H\right)} \geq \lambda_0.$$

Here, $H_{s_d:s_d+m}^{\mathrm{down}} = H_{s_d}^{\mathrm{down}}$ denotes $H_i^{\mathrm{down}} = H_{s_d}^{\mathrm{down}}$, $\forall i = s_d, s_d$ + 1, ..., $s_d$ + m , and $\lambda_0$ is a predetermined threshold. H in the summation refers to summing over H =F, M, FM, F1,F2 and $AD_{sd:sd+m}$ denotes $\{AD_{sd}, AD_{sd+1}, ..., AD_{sd+m}\}$. $P(\cdot|\cdot)$ refers to the conditional probability, specifically, the probability of the observed AD values at the $s_d$-th SNP, conditioned on the knowledge of the inherited chromosome from both parents at the $s_d$-th SNP as well as the labels of nearby SNPs. The conditional probability is calculated in exactly the same way as the recursion elaborated on in the next section. For each central SNP, the hyplotype state for at most 10 SNPs upstream or downstream is inferred.

Finally, as a result of the searching procedure, there will be at most two labels at each SNP. For those SNPs with less than two labels, a label is added as "Unknown". Then, the two labels of each SNP are merged to finalize the labeling procedure of LFLoH. According to the principle of final labeling, a SNP is labeled as F or M only after sufficient confidence that only paternal or maternal DNA fragments are detected, otherwise the SNP is labeled as FM. Specifically, the final label of the n-th SNP is

$$H_n = \begin{cases} \text{F} & \text{If } \{H_n^{\text{up}}, H_n^{\text{down}}\} = \{\text{F}^1\} \text{ or } \{\text{F}^1, \text{F}\} \text{ or } \{\text{F}^1, \text{FM}\} \\ & \quad \text{or } \{\text{F}^1, \text{Unknown}\} \text{ or } \{\text{F}_2\} \text{ or } \{\text{F}^2, \text{F}\} \text{ or } \{\text{F}^2, \text{Unknown}\} \\ \text{M} & \text{If } \{H_n^{\text{up}}, H_n^{\text{down}}\} = \{M\} \text{ or } \{\text{M}, \text{Unknown}\} \\ \text{FM} & \text{Otherwise} \end{cases}.$$

Step (b): Recursively updating the MCC rate and haplotype status of each SNP

The initial calculation of MCC rate in previous sections is performed based on SNPs with homozygous but different parental genotypes, and it is assumed that both paternal and maternal DNA fragments are detected at these SNPs. However, analyzing Large Fraction Loss of Haplotype (LFLoH) can provide new insights into whether parental DNA fragments are detected at each SNP, which can facilitate a re-estimation of MCC rate. With the new estimate of MCC rate, the likelihood will change and the final labels at all SNPs should be updated. Since the labels at SNPs are closely entangled with the MCC rate estimate, and both cannot be estimated at the same time, an iterative estimation method is adopted, whereby the estimate of each one is updated with the present estimate of the other one and this process is repeated until convergence. Under each stage, the MCC rate is first estimated using all SNPs labeled as FM, and then the LFLoH for each SNP is estimated using the new MCC rate estimate. This process is repeated until the difference between two adjacent estimates of MCC rate does not exceed a threshold (set to be 0.1 or 0.05) and the estimate in the final iterate is used as the final estimate of MCC rate and the corresponding LFLoH states are adopted as the final labels of haplotype status. If the MCC rate estimates do not converge after 10 rounds of iteration, the average of the 10 MCC rate estimates is then taken as the final estimate, and the haplotype status is updated using this estimate.

At least one advantage of the recursive procedure described herein is that it reduces the calculation of the likelihood function to the calculation of the recombination probability and the single-SNP likelihood. The computational load on the processors in the computer system is improved because of technological from the recursive proceure. In addition to a reduced processor load, the recursive procedure may result in fewer memory usage because of the consolidation equations being calculated. The recursive procedure described herein is novel and nonobvious, and is not well understood, routine, and conventional.

Step 7: Calculating the likelihood of observing the SNP data within regions adjacent to the disease-causing mutation site, combining the likelihood at each single SNP and the recombination probabilities.

The present disclosure provides a method of recursively calculating likelihood. Firstly, if the disease-causing mutation site is on the paternal chromosome, all SNPs whose final haplotype status is labeled as F or FM and where the paternal genotype is heterozygous are selected for recursion. If the disease-causing mutation site is on the maternal chromosome, all SNPs whose final haplotype status is labeled M or FM and where the maternal genotype is heterozygous are selected. The disease-causing mutation site is still denoted as the 0-th SNP. Among all retained SNPs downstream of it have ordinal numbers 1, 2, ..., $N_2$, while the upstream SNPs have ordinal numbers -1,-2, ... - $N_1$. For the i-th SNP, it is denoted

$$F_i = \begin{cases} 1 & \text{If the embryonic chromosome inherited from the father was actually from the grandfather} \\ 2 & \text{If the embryonic chromosome inherited from the father was actually from the grandmother} \end{cases},$$

$$M_i = \begin{cases} 1 & \text{If the embryonic chromosome inherited from the mother was actually from the grandfather} \\ 2 & \text{If the embryonic chromosome inherited from the mother was actually from the grandmother} \end{cases}$$

$AD_i$ is denoted as the Allele of Depth (AD) at the i-th SNP and $AD_{i:j} = \{AD_i, AD_{i+1}, ..., AD_{i+j}\}$ for i < j. Since given the genotype and haplotype labels at the disease-causing mutation site, the recombination and sequencing data in the upstream and downstream are conditionally independent, and the upstream and downstream analysis are dealt with separately. Here, formulating the recursion for the downstream region is further described. The probability that the parental embryonic chromosome is inherited from the grandfather or grandmother and the maternal embryonic chromosome is inherited from grandfather or grandmother is calculated, conditioned on the observation of all detected AD values and the inferenced haplotype status for SNPs within the adjacent region. Specifically, the goal is to calculate

$$\mathbf{P}\left(\mathbf{F_0} = i, \mathbf{M_0} = j \middle| \mathrm{AD}_{0:N_2}, \mathrm{H}_{0:N_2}\right) \text{ for } i, j \in \{1, 2\}.$$

The following Bayesian formula is utilized

$$\mathrm{P}\left(F_0 = i, M_0 = j | AD_{0:N_2}, H_{0:N_2}\right) \propto \mathrm{P}(F_0 = i, M_0 = j)\mathrm{P}\left(AD_{0:N_2} | F_0 = i, M_0 = j, H_{0:N_2}\right)$$

with non-informative prior:

$$\mathbf{P}(\mathbf{F_0} = i, \mathbf{M_0} = j) = \frac{1}{4} \quad \text{for } i, j \in \{1, 2\}.$$

The next step is to recursively calculate $P(AD_{0:N2}|F_0 = i, M_0 = j, H_{0:N2})$.

$$\mathrm{P}\left(AD_{n:N_2} | F_n = i, M_n = j, H_{n:N_2}\right)$$

$$= L_{ij}^{(n)} \sum_{k,l=1}^{2} \mathrm{P}\left(AD_{n+1:N_2} | F_{n+1} = k, M_{n+1} = l, H_{n+1:N_2}\right) \mathrm{P}(F_{n+1} = k, M_{n+1} = l | F_n = i, M_n = j)$$

The single-SNP likelihood is $L_{ij}^{(n)} = \mathrm{P}(AD_n | F_n = i, M_n = j, H_n)L_{ij}^{(N_2)}$ and the initial value is $L_{ij}^{(N_2)}$.

$$P(F_{n+1} = k, M_{n+1} = l | F_n = i, M_n = j)$$

is the recombination probability.

The recursive procedure described above reduces the calculation of the likelihood function to the calculation of the recombination probability and the single-SNP likelihood. The estimated recombination probability between any two positions on the human genome is obtained from a high-precision database of recombination rates as described in Kong et al. [2002]. The single-SNP likelihood has been described in step (5).

Step 8): Determining whether the embryo carries the disease-causing chromosome, and providing a level of confidence.

After calculating the likelihood, it is determined which one of the father's two chromosomes is inherited to the embryo and the same for the mother's chromosome by calculating Log Likelihood Ratio (LLR). If the disease-causing mutation site is on the paternal chromosome, the following is defined

$$\mathrm{LLR} = \log\left(\frac{\sum_{j=1}^{2} \mathbb{P}\left(\mathrm{AD}_{0:N} \middle| \mathrm{F}_0 = 1, \mathrm{M}_0 = j\right)}{\sum_{j=1}^{2} \mathbb{P}\left(\mathrm{AD}_{0:N} \middle| \mathrm{F}_0 = 2, \mathrm{M}_0 = j\right)}\right);$$

otherwise we defined

$$\mathrm{LLR} = \log\left(\frac{\sum_{i=1}^{2} \mathbb{P}\left(\mathrm{AD}_{0:N} \middle| \mathrm{F}_0 = i, \mathrm{M}_0 = 1\right)}{\sum_{i=1}^{2} \mathbb{P}\left(\mathrm{AD}_{0:N} \middle| \mathrm{F}_0 = i, \mathrm{M}_0 = 2\right)}\right).$$

A positive LLR indicates that the data considered favors that the allele is inherited from the father of the disease-carrying parent. Otherwise, the data supports that it is inherited from the mother of the disease-carrying parent.

However, it is recognized that how many SNPs to use for the calculation of likelihood cannot be determined in advance. If a certain fixed number of SNPs are used for the calculation, or if all SNPs within a certain fixed physical distance to the disease-causing mutation site are used for the calculation, it cannot be determined with certainty whether the information implied by these SNPs is enough to support conclusions and to what extent the likelihood varies with the number of used SNPs.

Therefore, the log-likelihood ratio curves are plotted as a function of the number of used SNPs and the property of this curve is exploited to determine whether the embryo carries the disease-causing mutation site. SNPs are successively added into the calculation of likelihood and for each $N_2$ = 1, 2, ..., the likelihood is calculated based on which parent's chromosome the disease-causing mutation site is on and the result is denoted as $LLR_{N_2}$. The graph of $LLR_{N_2}$ is then plotted against the physical distance of the $N_2$-th SNP to the disease-causing mutation site. Ideally, when the number of used SNPs is small, the data itself contains insufficient information about whether the embryo carries the disease-carrying allele, so the absolute value of the log-likelihood ratio is expected to be small and the curve should be close to the horizontal axis. As the number of used SNPs increases, more and more information is taken into consideration, and if the internal consistency of the data is good enough, the curve will rise or fall to a plateau with a sufficiently large absolute value. To make the log-likelihood curve more stable, the log-likelihood change for each pair of adjacent SNPs is limited to no more than 5. The log-likelihood value of the SNP for adjacent SNP pairs is clipped with log-likelihood change larger than this specified threshold.

Finally, when the number of used SNPs is large enough to determine whether the embryo carries the disease-carrying allele, adding new SNPs can hardly change the log-likelihood ratio anymore. Therefore, when the vertical coordinates of the curve tend to be stable, the sign of the stable value can be used as a criterion to determine whether the embryo carries the disease-carrying allele, and its absolute value serves as a measure for the confidence level. In practice, the Steady Log Likelihood Ratio (SLLR) downstream of the disease-causing mutation site is defined as

$$\text{SLLR}_{\text{down}} = \frac{1}{N_0} \sum_{n=n_0}^{N_0+n_0} \widehat{\text{LLR}}_n,$$

where

$$n_0 = \max\left\{ n : \left( \max_{n \le i \le n+N_0} \widehat{\text{LLR}}_i - \min_{n \le i \le n+N_0} \widehat{\text{LLR}}_i \right) \le \varepsilon_0 \right\}.$$

Here, $\varepsilon_0$ is a predetermined threshold. In experiments, $\varepsilon = 0.1$. Here, $N_0$ is the number of SNPs needed to identify the stability, which is by default 30. If there is no consecutive 30 SNPs whose log-likelihood range is within 0.1, then $N_0$ is reduced to 10 to detect whether the curve stabilizes. If either upstream or downstream region has less than 10 SNPs in the data, this region is ignored and the confidence interval according to the curve on the other side is determined. This happens when the disease-causing mutation site is at either end of the chromosome, or near the spindles. Similarly, we compute the upstream steady log likelihood SLLRup and denote their arithmetic average as Average Steady Log-Likelihood Ratio (ASLLR).

According to SLLR, ASLLR, and other properties of the log-likelihood curve, the confidence level of judgements is classified into four categories: Highly Confident, Moderately Confident, Possible, and Undetermined. Specifically, if SLLRup•SLLRdown < 0 for a sample, the sample is classified into "Undetermined" directly, since the opposite conclusion is obtained from the upstream and downstream regions. For the remaining samples, the Maximally Different Log-Likelihood Ratio (MDLLR) is defined as:

$$\text{MDLLR} := \begin{cases} \min\left\{ \min\left\{ \widehat{\text{LLR}}_n : -N_1 \le n \le N_2 \right\}, 0 \right\} & \text{If } \text{SLLR}_{\text{up}} > 0, \text{SLLR}_{\text{down}} > 0 \\ -\max\left\{ \max\left\{ \widehat{\text{LLR}}_n : -N_1 \le n \le N_2 \right\}, 0 \right\} & \text{If } \text{SLLR}_{\text{up}} < 0, \text{SLLR}_{\text{down}} < 0 \end{cases}.$$

Finally the confidence level is determined by

- Highly Confident: If ASLLR > 4, MDLLR = 0, and $|\text{SLLR}_{\text{up}}| > 1$, $|\text{SLLRd}_{\text{own}}| > 1$, $N_0 = 30$.
- Moderately Confident: If ASLLR $\le$ 4, MDLLR = 0, $|\text{SLLR}_{\text{up}}| > 1$, $|\text{SLLR}_{\text{down}}| > 1$, $N_0 = 30$; or ASLLR > 4, MDLLR $\ge$ -5, $N_0 = 30$.
- Possible: If none of the statements above holds and the sample is not categorized as 'undetermined'.

Following the above protocol, the genotype and which allele is inherited from parents at any single SNP can be determined. This information can then be used to execute the disease carrying analysis for the monogenic disease, and combine multiple identified SNPs of the embryo to determine its susceptibility of a polygenic disease.

The present invention also refers to a method of analyzing the chromosome aneuploidy via a biological sample of culture medium of an in vitro cultured embryo to do preimplantation genetic testing for aneuploidy.

## COMPUTER SYSTEM

Any of the computer systems mentioned herein may utilize any suitable number of subsystems. In some embodiments, a computer system includes a single computer apparatus, where the subsystems can be the components of the computer apparatus. In other embodiments, a computer system can include multiple computer apparatuses, each being a subsystem, with internal components.

A computer system can include a plurality of the same components or subsystems, e.g., connected together by external interface or by an internal interface. In some embodiments, computer systems, subsystems, or apparatuses can communicate over a network. In such instances, one computer can be considered a client and another computer a server, where each can be part of a same computer system. A client and a server can each include multiple systems, subsystems, or components.

It should be understood that any of the embodiments of the present invention can be implemented in the form of control logic using hardware (e.g. an application specific integrated circuit or field programmable gate array) and/or using computer software with a generally programmable processor in a modular or integrated manner. Based on the disclosure and teachings provided herein, a person of ordinary skill in the art will know and appreciate other ways and/or methods to implement embodiments of the present invention using hardware and a combination of hardware and software. Any of the software components or functions described in this application may be implemented as software code to be executed by a processor using any suitable computer language such as, for example, Java, C++ or Perl using, for example, conventional or object-oriented techniques. The software code may be stored as a series of instructions or commands on a computer readable medium for storage and/or transmission, suitable media include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices. Such programs may also be encoded and transmitted using carrier signals adapted for transmission via wired, optical, and/or wireless networks conforming to a variety of protocols, including the Internet. As such, a computer readable medium according to an embodiment of the present invention may be created using a data signal encoded with such programs. Computer readable media encoded with the program code may be packaged with a compatible device or provided separately from other devices (e.g., via Internet download). Any such computer readable medium may reside on or within a single computer program product (e.g. a hard drive, a CD, or an entire computer system), and may be present on or within different computer program products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective steps or a respective group of steps. Although presented as numbered steps, steps of methods herein can be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

## EXAMPLES

The invention will now be illustrated, but not limited, by reference to the specific embodiments described in the following examples.

### Example 1: Study Subjects

The first case was an autosomal dominant genetic disease. The wife has a family history of an autosomal dominant disorder and has suffered from hereditary osteogenesis imperfecta (OI), which is characterized by easy bone fracture without obvious cause or minimal injury. The couple already had an affected son who also suffers from OI. The genetic diagnosis of the wife showed a deletion c.299-14_c.302del at the COL1A1 (collagen, type I, alpha 1) gene, already known to cause this disease. This couple underwent PGT-M treatment. Eighteen metaphase-II oocytes were collected and fertilized by intra-cytoplasmic sperm injection (ICSI). Twelve embryos developed to the blastocyst stage, and several trophectoderm cells were biopsied from each embryo for PGT-M. The second case was an autosomal recessive disease. Both parents carried a deafness recessive heredity disorder. The genetic diagnosis of the wife showed an insertion c.99_100insT at the USH2A gene. The genetic diagnosis of the husband showed a duplication c.991dupA and a mutation c.5699G>T at the USH2A gene. The couple already had a deaf daughter whose genetic diagnosis showed the insertion c.99_100insT, the duplication c.991dupA and the mutation c.5699G>T at the USH2A gene that was inherited from her parents. These mutations cause the deafness. This couple underwent PGT-M treatment. Nine metaphase-II oocytes were collected and fertilized by ICSI. Four embryos developed to the blastocyst stage, and several trophectoderm cells were biopsied from each embryo for PGT-M.

**Blastocyst Biopsy and PGT-M Testing.** Standard protocols were used for ICSI. Embryos were cultured individually in 15-μL microdrops of the equilibrated Vitrolife G-series media with human serum albumin (HSA) (LifeGlobal) overlain with mineral oil in Miri incubators (ESCO) at 37 °C in a dry atmosphere of 5% O2, 6-7% CO2 balanced with N2. Embryos were evaluated on day 5 or 6 for Trophectoderm (TE) biopsy. A few TE cells from each hatching blastocyst were biopsied and sent to an outside PGT lab for PGT-M testing.

## Reference Example 2: Sample Collections

**Culture Medium Sample and Discarded Embryo Collection.** Immediately after the blastocysts were transferred to another dish for biopsy, 13-μL spent culture media were removed from each of the residual spent medium drops. The discarded embryos and their corresponding spent culture media samples were also collected for analysis. Each discarded embryo was gently moved with a pipette tip to the edge of its microdrop and then removed and transferred into a RNase-DNase-free PCR tube containing 3 μL of lysis buffer. Pipette tips were changed between collections of each sample to avoid cross-sample contamination. Media drops incubated and collected under identical conditions to those used for blastocyst culture, but never containing embryos, served as negative controls.

All samples were immediately frozen after collection and stored at -80 °C until analyzed.

## Reference Example 3: Protocol for Lysis

**Spent Culture Medium Lysis.** The frozen 13-μL spent culture medium samples were thawed and gently mixed, and 10.8 μL was removed to a PCR tube (Maximum Recovery, Axygen) containing 1.2 μL 10x lysis buffer (1x lysis buffer: 60 mM Tris-Ac pH 8.3, 2 mM EDTA pH 8.0, 15 mM DTT, 0.5 μM carrier ssDNA (5'-TCAGGTTTTCCTGAA-3', Thermo Fisher Scientific oligo with PAGE purification)), spun down. It was heated at 75°C for 30 min. Then incubated at 75°C for 30 min. 0.5 μL 35 mg/mL QIAGEN protease (dissolved in water and stored at 4°C) was added, spun down, and incubated at 55°C for 2 hrs followed by 80°C for 30 min. The resulting lysate in PCR tubes could be subject to modified linear transposon-based amplification as described herein immediately, or stored in a freezer for later use.

**Discarded Embryo Lysis.** The frozen discarded embryos were thawed and heated at 75°C for 30 min. Then the discarded embryo samples were lysed by adding 0.5 μL 5mg/mL Qiagen Protease and heating (2 h at 55 °C and 30 min at 80 °C) in 3 μL of lysis buffer.

## Example 4: Whole Genome Amplification

**Transposome preparation.** Transposon DNA (5'/Phos/CTGTCTCTTATACACATCTGAACAGAATTTAATAC GACTCACTATAGGGAGATG TGTATAAGAGACAG-3', Thermo Fisher Scientific oligo with PAGE purification) was annealed by gradual cooling in annealing buffer (20 mM Tris-Ac pH 8.3, 50 mM NaCl, 2 mM EDTA pH 8.0) into a 1.5 μM self-looping structure. The 1.5 μM annealed transposon DNA was then mixed with an equal volume of ~1 μM Tn5 transposase (Lucigen, EZ-Tn5™ Transposase) and incubated at room temperature for 30 min, dimerizing into transposomes with a final concentration of ~0.25 μM. The transposomes can be stored at -20°C for a long time, and each single-cell transposon-based amplification needs ~0.5μL transposome.

**Whole genome amplification by modified LIANTI (Linear Amplification via Transposon Insertion) method.** Starting with the culture medium lysate, a 20 μL transposition mixture was assembled in the buffer containing 2.5 mM $MgCl_2$ and 18.75 nM transposome. The transposition reaction was carried out at 55°C for 12 min. Then 0.84 μL mixture of EDTA and ssDNA was added to each tube and was incubated at 68 °C for 30 min. After removing transposase reaction, 0.2 μL Q5 HF DNA Polymerase (New England Biolabs) was added and was heated at 73°C for 45 seconds in the presence of 2 mM $MgCl_2$ and 200 μM dNTPs for fragments end filling and extension. 0.5 μL 4mg/mL Qiagen protease was added and the PCR tube was heated at 50 °C for 1 hour in the presence of 4 mM EDTA to inactivate DNA polymerase, followed by protease heat inactivation by 77°C for 20 min in the presence of 450 mM NaCl in a total volume of ~25 μL. DNA fragments were amplified to RNAs in a 90 μL T7 in vitro transcription reaction mixture overnight at 37 °C as described in in US 10,894,980 and C Chen, D Xing, L Tan, H Li, G Zhou, L Huang, XS Xie
Science 2017; 356 (6334): 189-194

The next day, 10 μL 0.5M EDTA was added to each tube and RNAs were column purified (Zymo Research). 18 μL RNAs were transferred to a PCR tube containing 3.1 μL mixture of 6.5mM each dNTPs, 9.7 μM carrier ssDNA (5'-TCAGGTTTTCCTGAA-3', Thermo Fisher Scientific oligo with PAGE purification) and 3.2 U/μL SUPERase In RNase Inhibitor (Invitrogen). The denaturation incubation was at 70 °C 1min, 90 °C 15s, and followed by ice quenching. The reverse transcription reaction for the first stand was carried out in a 30 μL SuperScript IV reserve reaction system including 0.67 mM each dNTPs, 0.6 U/μL SUPERase In RNase Inhibitor (Invitrogen) and 6 U/μL SuperScript IV Reserve Transcriptase (Invitrogen) in SuperScript IV buffer, with the primer 5'-AGATGTGTATAAGAGACAG-3', Thermo Fisher Scientific oligo with PAGE purification, and the incubation program was 25°C 1 min, 37°C 1 min, 42°C 1 min, 50°C 1 min, 55°C 15 min, 60°C 10 min, 65°C 12 min, 70°C 8 min, 75°C 5 min, 80°C 10 min. RNA was then removed by incubation at 37°C for 30 min with 10 ng/μL affinity-purified RNase A (Invitrogen) and 0.08 U/μL RNase H (New England Biolabs). Second strand synthesis was carried out in a 100 μL Q5 DNA polymerase system (New England Biolabs, 1X Q5 reaction buffer, 1X Q5 High GC enhancer, 200 μM dNTPs, 0.5 μM primer, 0.02 U/μL Q5 DNA polymerase), with the primer 5'-NNNNNNNNGGGAGATGTGTATAAGAGACAG-3', Thermo Fisher Scientific oligo with PAGE purification. Each tube was heated at 98°C for 30 s, then using 10 cycles of 10 s at 98°C, 30s at 58°C, 30s at 60°C, 30s at 65°C, 2.5 min at 70°C, then 72°C 6 min for strand extension. The resulted amplicons were column purified into 23 μL elution buffer and stored at -20°C.

The DNA concentration of the product after amplification was measured using a Qubit 2.0 fluorometer (Thermo-Fisher Scientific) with the Qubit dsDNA HS Assay kit (Life Technologies). In the first case, 17 spent culture media were amplified successfully. In the second case, 9 spent culture media were amplified using this method. 8 culture media were amplified successfully, but one failed. The amplification success rate of the spent culture medium was 96.15%.

**Discarded Embryo DNA Amplification.** DNA was amplified following the LIANTI amplification method described in US 10,894,980 and C Chen, D Xing, L Tan, H Li, G Zhou, L Huang, XS Xie. Science 2017; 356 (6334): 189-194, except the DNA primer 19_1 5'-AGATGTGTATAAGAGACAG-3', Thermo Fisher Scientific oligo with PAGE purification was added in the reaction of the first strand cDNA synthesis. Four discarded embryos in the first case and five discarded embryos in the second case were all amplified successfully.

**Example 5: Comparison of Variables in the Lysis and Amplification Methods**

A diploid human cell line, BJ primary human foreskin fibroblast (ATCC CRL-2522), was used as described in the LIANTI paper (Chen 2017) and the comparison paper of single-cell whole genome amplification methods (Huang 2015). The BJ cell line was used as a standard sample for different lysis and amplification methods' comparison. In order to compare the performance of different amplification methods on spent culture medium, the BJ cell line was cultured in Eagle's minimum essential medium (ATCC) supplemented with 10% fetal bovine serum, 100 I.U./mL penicillin and 100 μg/mL streptomycin in 5% $CO_2$ 37°C incubator. After 48 hours, the culture medium of BJ cell line was collected. Then BJ cells were trypsinized and washed in 1X PBS, followed by resuspension into 1X PBS. Single BJ cells were then mouth-pipetted into PCR tubes containing 3 μL lysis buffer.

The LIANTI procedure that described in US 10,894,980 and C Chen, D Xing, L Tan, H Li, G Zhou, L Huang, XS Xie. Science 2017; 356 (6334): 189-194 was used to amplify three single cells and three culture medium samples, employing 0.5 mg/ml (working concentration) QIAGEN protease, 6.25 nM (working concentration) transposome. No DNA was successfully amplified.

Then, the amplification method described in Examples 3-4 was used to amplify culture medium DNA, except adjusting a single variable each time. Particularly, the variables include the working concentration of the protease, the working concentration of the transposome, the PCR cycles and the primer sequence. Three repetitions were performed per variable.

The comparison results were shown in Table 1 and Fig. 3. When 0.5 mg/ml (working concentration) QIAGEN protease or 6.25 nM (working concentration) transposome was used in combination with DNA primer 19_1, considerable DNA can be successfully amplified. When the working concentration of QIAGEN protease or transposome was increased, the amplified DNA amount increased accordingly.

When no DNA primer was added (wo), as with the LIANTI procedure described in US 10,894,980 and C Chen, D Xing, L Tan, H Li, G Zhou, L Huang, XS Xie. Science 2017; 356 (6334): 189-194, no DNA was successfully amplified. When primer 19_1, 19_2, 15_1 or random primer 3+14N with specific sequence was added, however, considerable DNA can be successfully amplified. The amplification result of primer 25_1 is not as good as that of primer 19_1 or 19_2, probably due to the increasing mismatch of primer 25_1 with the RNA strand of the sequence of Tn5 transposition binding site. Preferably, the length of primer is 15bp to 20bp. As compared with 3+14N, when random primer 5+14N was added, no DNA was successfully amplified, verifying the importance of the 3'-end sequence of the DNA primer.

Table 1

| Protocol | Sample types | Variables types | Variables information | rep1 (DNA amount ng) | rep2 (DNA amount ng) | rep3 (DNA amount ng) |
|---|---|---|---|---|---|---|
| Original LIANTI Protocol | Single cell | \ | \ | 3.42 | 2.31 | 0 |
| | Culture medium | \ | \ | 0 | 0 | 1.5 |

(continued)

| Protocol | Sample types | Variables types | Variables information | rep1 (DNA amount ng) | rep2 (DNA amount ng) | rep3 (DNA amount ng) |
|---|---|---|---|---|---|---|
| Amplification Method of the Present Invention | Culture medium | Protease Working Concentration | 0.16 mg/ml | 36.3 | 16.5 | 61.8 |
| | | | 0.6 mg/ml | 103.5 | 98.4 | 51.6 |
| | | | 1 mg/ml | 132.6 | 315 | 282 |
| | | | 1.4 mg/ml | 74.7 | 133.5 | 53.4 |
| | | | 1.8 mg/ml | 117.6 | 89.4 | 149.7 |
| | | Transposome Working Concentration | 6.25 nM | 106.8 | 11.775 | 65.1 |
| | | | 12.5 nM | 64.8 | 72.3 | 113.4 |
| | | | 18.75 nM | 171 | 105 | 156 |
| | | | 37.5 nM | 369 | 1197 | 1308 |
| | | Primer | 15_1 | 49.2 | 26.55 | 40.8 |
| | | | 19_1 | 78.3 | 116.1 | 48.3 |
| | | | 19_2 | 63.6 | 84.6 | 88.8 |
| | | | 25_1 | 10.68 | 17.1 | 3.84 |
| | | | 3+14N | 44.7 | 30.3 | 26.55 |
| | | | 5+14N | 2.34 | 3.54 | 1.8 |
| | | PCR cycles | 1 cycle | 1.56 | 0 | 10.14 |
| | | | 4 cycles | 0 | 2.97 | 5.52 |
| | | | 8 cycles | 53.7 | 23.4 | 9.21 |
| | | | 10 cycles | 100.8 | 104.7 | 73.2 |
| | | | 12 cycles | 178.5 | 130.8 | 92.1 |
| | | | 14 cycles | 459 | 678 | 78.3 |

**Example 6: Library Preparation for Sequencing**

Upon library preparation, the amplicons were subject to a conventional sonication process (Covaris S2) to the length required by sequencing platforms. The final insert size was suitable for 2X150 bp pair-end Illumina sequencing. Library preparation was performed by NEBNext Ultra II DNA Library Prep Kit for Illumina (New England Biolabs), following the instructions of the manufacturer and skipping the optional size selection step. For the gDNA of blood samples, all the samples were subject to a conventional sonication process (Covaris S2). Library preparation was performed by a PCR-free Library Prep Kit, NEBNext Ultra II DNA Library Prep Kit for Illumina (NEB #E7645S/L).

**Illumina sequencing.** The bulk samples, culture medium samples, and discarded embryo samples were sequenced on the Illumina NovaSeq 6000 System or Illumina HiSeq HiSeq 4000 platform with 2X150 bp pair-end sequencing. Sequencing lanes were shared by 24 samples with NEBNext Indexes. Each gDNA sample was sequenced to generate ~90 Gb raw data and each culture medium sample or discarded embryo was sequenced to generate ~30 Gb raw data.

**Example 7: Mapping and SNP Calling**

The genome coverage and allele dropout rate of each sample was calculated. Although the LIANTI process that is described in US 10,894,980 and C Chen, D Xing, L Tan, H Li, G Zhou, L Huang, XS Xie. Science 2017; 356 (6334): 189-194 offers the highest genome coverage compared to other single-cell whole genome amplification methods (Chen et al 2017), the genome coverage of the DNAs in the spent culture medium exhibited very low genome coverage and a high allele dropout rate. In the first case, the genome coverage of the 17 spent culture media varied from 16.38% to 70.34%, as shown in Fig. 4. In the second case, the genome coverage of the 9-success amplified spent culture media varied from 4.79% to 34.97%, as shown in Fig. 5. Their ADO rate was much higher than 5%. The genome coverage was far from reaching the standard that allows linkage analysis with several loci. (ESHRE 2020).

**Example 8: Linkage Analysis by Bayesian and Disease Carrying Analysis**

The Dry Lab method described above was utilized for linkage analysis.

In the first case, the heterozygous SNPs in chromosome 17 of the wife and the homozygous SNPs of the husband were the subject of analysis. In Fig. 6, the linkage analysis plots for four SCMs in Case 1 are shown. Among these four SCMs, 1-SCM-02 had the highest and tightest number of SNP loci, so in determining the absence of the inherited result of the mother's disease-causing allele in the embryo, the corresponding confidence level was 'High Confidence'. In 1-SCM-05 and 1-SCM-07, although large fractions of haplotypes were lost, the detected SNPs were calculated by Bayesian models to support that the disease-causing allele was inherited in the embryos with 'High Confidence' level of the disease-carrying analysis. In contrast, in 1-SCM_06, fewer SNP loci were detected than in the other three SCMs, a 'Moderate Confidence' level of the disease-carrying analysis in determining the absence of an inherited disease-causing allele.

For the second case, to carry out the linkage analysis of each embryo, the genomes of the blood samples of the wife, husband, and disease-carrying daughter were sequenced. All SNPs of the three individuals were called with a sequencing depth >10x within 2 Mb of the USH2A gene. The heterozygous SNPs in chromosome 1 of the wife and homozygous SNPs of the husband for the mutation site (c.99_100insT) that is carried by the wife were the focus of analysis, and the heterozygous SNPs in chromosome 1 of the husband and homozygous SNPs of the wife for the mutation sites (c.991 dupA and c.5699G>T) that is carried by the husband were the focus of analysis. The results for all spent culture medium in cases 1 and 2 are summarized in Table 2.

Table 2

| Case | Chrom | Pos | Disease-carring Parent | Disease Allele | Sample Name | Sample Type | Rate of GQ <= 3 | GQ Threshold | err_rate(10 | #of SNP /common SNPs | init_mcc_rate (10M) | final_mcc rate (10M) | Allele Classification | Truth | Classification |
|---|---|---|---|---|---|---|---|---|---|---|---|---|---|---|---|
| 1 | 17 | 48276965 | Mather | 1 | 1-SCM-01 | SCM | 0.159 | 6 | 0.014 | 0.037 | 0.296 | 0.085 | 2 | | High Confidence |
| | | | | | 1-SCM-02 | SCM | 0.124 | 6 | 0.009 | 0.038 | 0.131 | -0.087 | 2 | 2 | High Confidence |
| | | | | | 1-SCM-03 | SCM | 0.131 | 6 | 0.007 | 0.012 | 0.318 | 0.3 | 2 | 2 | Undetermined |
| | | | | | 1-SCM-04 | SCM | 0.173 | 6 | 0.015 | 0.039 | 0.031 | 0.054 | 2 | 2 | Moderate Confidence |
| | | | | | 1-SCM-05 | SCM | 0.114 | 6 | 0.007 | 0.127 | 0.032 | 0.064 | 1 | 1 | Moderate Confidence |
| | | | | | 1-SCM-06 | SCM | 0.144 | 6 | 0.011 | 0.018 | 0.331 | -0.315 | 2 | 2 | Possible |
| | | | | | 1-SCM-07 | SCM | 0.149 | 6 | 0.013 | 0.019 | 0.278 | 0.33 | 1 | 1 | High Confidence |
| | | | | | 1-SCM-08 | SCM | 0.145 | 6 | 0.014 | 0.033 | -0.076 | -0.002 | 1 | 1 | Possible |
| | | | | | 1-SCM-10 | SCM | 0.148 | 6 | 0.015 | 0.028 | 0.126 | 0.019 | / | 2 1 | Undetermined |
| | | | | | 1-SCM-12 | SCM | 0.099 | 6 | 0.009 | 0.02 | -0.53 | -0.108 | / | 2 | Rule Out |
| | | | | | 1-SCM-14 | SCM | 0.124 | 6 | 0.01 | 0.022 | 0.025 | -0.063 | 1 | 1 | High Confidence |
| | | | | | 1-SCM-15 | SCM | 0.149 | 6 | 0.043 | 0.008 | 0.663 | 0.365 | / | 2 | Rule Out |
| | | | | | 1-SCM-16 | SCM | 0.158 | 6 | 0.01 | 0.039 | -0.057 | 0.044 | 1 | 1 | High Confidence |
| | | | | | 1-SCM-11 | SCM of Discaded Embryo | 0.154 | 6 | 0.014 | 0.041 | 0.083 | 0.048 | 1 | 1 | High Confidence |
| | | | | | 1-SCM-13 | SCM of Discaded Embryo | 0.147 | 6 | 0.013 | 0.033 | 0.051 | -0.002 | 1 | 1 | Possible |
| | | | | | 1-SCM-17 | SCM of Discaded Embryo | 0.123 | 6 | 0.012 | 0.036 | 0.081 | 0.067 | 2 | 2 | High Confidence |
| 2 | 1 | 216595579 | Mather | 1 | 2-SCM-01 | SCM | 0.105 | 6 | 0.016 | 0.021 | 0.208 | -0.219 | 2 | | High Confidence |
| | | | | | 2-SCM-04 | SCM | 0.097 | | 0.011 | 0.003 | 0.256 | 0.605 | / | 1 | Possible |
| | | | | | 2-SCM-05 | SCM | 0.086 | 6 | 0.009 | 0.01 | 0.003 | -0.139 | 1 | 1 | Moderate Confidence |

| Case | Chrom | Pos | Disease-carring Parent | Disease Allele | Sample Name | Sample Type | Rate of GQ <= 3 | GQ Threshold | err_rate(10 | #of SNP /common SNPs | init_mcc_rate (10M) | final_mcc rate (10M) | Allele Classification | Truth | Classification |
|---|---|---|---|---|---|---|---|---|---|---|---|---|---|---|---|
| | | | | | 2-SCM-02 | SCM of Discaded Embryo | 0.122 | 6 | 0.011 | 0.005 | 0.313 | 0.313 | 2 | 2 | Possible |
| | | | | | 2-SCM-03 | SCM of Discaded Embryo | 0.144 | 9 6 | 0.017 | 0.009 | 0.085 | -0.502 | / | 1 | Rule Out |
| | | | | | 2-SCM-06 | SCM of Discaded Embryo | 0.129 | 6 | 0.016 | 0.01 | -0.002 | -0.362 | 2 | 2 | Undetermined |
| | | | | | 2-SCM-07 | SCM of Discaded Embryo | 0.116 | 6 | 0.019 | 0.011 | 0.195 | -0.423 | 1 | 1 | High Confidence |
| | | | | | 2-SCM-09 | SCM of Discaded Embryo | 0.18 | 6 | 0.012 | 0.002 | -0.409 | 0.01 | / | 1 | Undetermined |
| 1 | 216498799 | Father | 1 | | 2-SCM-01 | SCM | 0.105 | | 0.016 | 0.021 | 0.208 | -0.219 | 2 | | Moderate Confidence |
| | | | | | 2-SCM-04 | SCM | 0.097 | 9 | 0.011 | 0.032 | 0.256 | 0.605 | 1 | 1 | Possible |
| | | | | | 2-SCM-05 | SCM | 0.086 | 6 | 0.009 | 0.01 | 0.003 | -0.139 | 1 | 1 | Moderate Confidence |
| | | | | | 2-SCM-02 | SCM of Discaded Embryo | 0.122 | 6 | 0.011 | 0.005 | 0.313 | 0.313 | 1 | 2 1 | Possible |
| | | | | | 2-SCM-05 | SCM of Discaded Embryo | 0.144 | 6 | 0.017 | 0.009 | 0.085 | -0.502 | 1 | 1 | Rule Out |
| | | | | | 2-SCM-06 | SCM of Discaded Embryo | 0.129 | 6 | 0.016 | 0.01 | -0.002 | -0.362 | 1 | 1 | High Confidence |
| | | | | | 2-SCM-07 | SCM of Discaded Embryo | 0.116 | 6 | 0.019 | 0.011 | 0.195 | -0.423 | 2 | 2 | High Confidence |

(continued)

| Case | Chrom | Pos | Disease-carring Parent | Disease Allele | Sample Name | Sample Type | Rate of GQ <= 3 | GQ Threshold | err_rate(10 | #of SNP /common SNPs | init_mcc_rate (10M) | final_mcc rate (10M) | Allele Classification | Truth | Classification |
|------|-------|-----|------------------------|----------------|-------------|-------------|-----------------|--------------|-------------|---------------------|--------------------|---------------------|----------------------|-------|----------------|
|      |       |     |                        |                | 2-SCM-09    | SCM of Discaded Embryo | 0.18 | 6 | 0.012 | 0.002 | -0.409 | 0.01 | 1 | 1 | Possible |

For the first case, the culture medium for discarded embryo 1-SCM-11 was omitted due to the lack of a reliable standard for that sample, since the corresponding discarded embryo was not collected. Among the remaining 16 culture media, two were excluded because of unusual maternal cell contamination (MCC) rates. Both MCC rates were outside the normative range (1-SCM-12 had a rate of -0.53, and 1-SCM-15 had a rate of +0.663) prior to calibration, leading to their elimination from subsequent disease-carrying analysis. The only culture medium deemed 'undetermined' in instance 1 was 1-SCM-10, attributed to the divergent signs of the stationary log-likelihood ratio on either side of the disease-causing mutation site. In the upstream, the log-likelihood ratio was convergent around +10.1 and that of the downstream region converged to a value around -11.3. Despite this, disease-carrying analysis was carried out for 13 samples, classifying 8 as high confidence, 2 as moderate confidence, and 3 as possible. These results agreed with the biopsy examination.

For the second case, all 14 samples (7 culture media for both parental sides) are counted into the final result. 2-SCM-03 was disqualified in the quality control stage due to its abnormal MCC rate (-0.501) after calibration, precluding it from further analysis for both disease-causing mutation sites.

Moreover, 2-SCM-04 (maternal side) and 2-SCM-08 (maternal side) were labeled 'undetermined,' indicating an uncertain result about whether the inherited gene from the mother was disease-causing. Of the remaining 10 samples (6 paternal and 4 maternal), 3 were categorized as 'highly confident,' 2 as 'moderate confident,' and 5 as 'possible.' Again, these results were consistent with those from biopsy examinations.

As an illustration, the culture medium 1-SCM-01 in the first case was the subject of analysis. Qualified SNPs were selected within a region extending 10Mbp upstream and downstream from the disease-causing mutation site, enabling the estimation of basic statistics like sequencing error rate and Maternal Cell Contamination (MCC) rate. This includes removing all SNPs with either parental allele depth lower than 5, or with QUAL value lower than 30. All SNPs with more than two alleles were removed. In this region, out of 74935 SNPs, 11913 had a Genotype Quality (GQ) value of 3 or lower. All SNPs with a GQ value of 6 or lower were eliminated, which corresponded to the lowest 20 percent of all SNP GP values. Among the remaining SNPs, 4911 SNPs were identified where both parents exhibited the same homozygous genotype (i.e., either both were 00 or both were 11). Within this subset, 220 SNPs exhibited at least one erroneous read. These 4911 SNPs generated a total of 45499 reads, with 629 erroneous reads, yielding an estimated sequencing error rate of 0.0138. On scrutinizing all qualified SNPs, 484 SNPs were identified wherein both parents displayed different but homogeneous genotypes (i.e., either the paternal genotype was 00 and the maternal genotype was 11, or vice versa). The total number of reads in these SNPs was 4779, with 3097 derived from the mother and the remainder from the father. This data yielded an MCC rate of 0.296, calculated as (3097*2-4779)/4779. Post calibration, 332 of these 484 SNPs were classified as either 'containing paternal chromosome only' or 'containing maternal chromosome only'. The remaining 152 SNPs, regarded as 'containing both parental chromosomes', were used to calculate the estimated MCC rate after calibration. These SNPs generated a total of 1761 reads, with 955 from the mother and the rest from the father, resulting in an estimated MCC rate of 0.0846. Using the calibrated MCC rate, the log-likelihood value was iteratively computed from the disease-causing mutation site. The log-likelihood ratio of any segment of SNPs, either upstream or downstream, was negative. From the 21st SNP upstream (the 48003174th base pair), the log-likelihood ratio stabilized around -15.1. Meanwhile, in the downstream region, from the 7th SNP (the 48321621st base pair), the log-likelihood ratio stabilized around -11.2. Based on this analysis, it was deduced that this sample did not inherit the disease-carrying gene from the mother and labeled this result as 'highly confident'. Comparison of the allele classification results of SCM with the results of TE-biopsy or discarded-embryo produced a consistent rate of 100%. The accuracy of this method described herein is high enough for PGT-M.

### Example 9: Disease-Carrying Analysis for PGT-P

To demonstrate the efficacy of the method described herein in determining the susceptibility of polygenic disease, the Polygenetic Risk Score (PRS) of type 2 diabetes was calculated for the second case. The PRS is derived from a combination of multiple genetic variants, specifically single nucleotide polymorphisms (SNPs), which are directly associated to the disease. From the 139 prevalent SNPs related to type 2 diabetes mentioned in Xue, et al. 2018, 57 SNPs were identified to be present in the parental samples of the family. For each SNP, a Bayesian model was employed separately for determining the genotype of each disease-related SNP. Possible scores for the SNPs are 0, 1, or 2, which represent homozygous non-effect allele, heterozygous allele, and homozygous effect allele, respectively. PRS is defined as the weighted sum of these scores, with the weight equal to the absolute value of the log odds ratio to the minor allele at each SNP. We examined three discarded embryos from the second case, along with their corresponding culture mediums. The results indicate an alignment and correlation between the PRS scores of the discarded embryos and those derived from the culture mediums, as elaborated in Table 3 and Fig. 7.

Table 3.

| Embryo Name | 2-Embryo-03 | 2-Embryo-07 | 2-Embryo-08 |
|---|---|---|---|
| PRS in the culture medium | 3.532 | 3.767 | 3.288 |
| PRS in the discarded embryo | 3.634 | 3.814 | 3.36 |

**Example 10: Non-Invasive Preimplantation Genetic Testing for Aneuploidy**

In the addition to the niPGT-M and niPGT-P, the sequencing data of the present invention is used for non-invasive preimplantation genetic testing for aneuploidy (niPGT-A). After high-quality reads were aligned to the human reference genome hg19, copy number variation (CNV) detection for each sample was performed. Mapped reads were subjected to correction of GC bias resulting from DNA's GC contents. The read counts for each bin of 1000kb were defined as the copy number (CN). CNV analysis was conducted using HMMcopy (v1.28.0) and DNAcopy (v1.58.0) at a resolution of 1Mb. Segmentation was performed via HMMcopy with an e value of 0.9999, and using DNAcopy with an alpha value of 0.0001. CNVs were identified by using the results from both HMMcopy and DNA to identify aneuploid segments larger than 10 Mb, while retaining double-positive counts as true events. The cytoband location of those aneuploid segments was calculated using the cytoband file for hg19 from the University of California, Santa Cruz, database (available at world wide website hgdownload.cse.ucsc.edu/downloads.html).

In the first case, the CN plots of the spent culture medium was compared with TE-biopsy. The CNV results of the spent culture medium were abnormal when the corresponding TE-biopsy results were abnormal. For example, in Fig. 8 (a), the niPGT-A result of 1-SCM-02 showed a monosomy 10 aneuploidy, which was consistant with the corresponding TE result. In Fig. 8 (b), the niPGT-A result of 1-SCM-04 showed chromosome loss on chromosome 9's q arm, which was also consistant with its TE-biopsy result. In Fig. 8 (c), the niPGT-A result of 1-SCM-14 was normal, which the corresponding TE-biopsy result was also normal. These results demonstrate that the method described herein could analyze the copy number of spent culture medium.

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

The patents, published applications, and scientific literature referred to herein establish the knowledge of those skilled in the art and are hereby incorporated by reference in their entirety to the same extent as if each was specifically and individually.

The specific details of particular embodiments may be combined in any suitable manner without departing from the spirit and scope of embodiments of the invention. However, other embodiments of the invention may be directed to specific embodiments relating to each individual aspect, or specific combinations of these individual aspects.

The sequences used in the present invention are summarized in Table 4.

Table 4

| Seq. ID No. | Description | Sequence Information |
|---|---|---|
| 1 | DNA Primer 19_1 | 5'-AGATGTGTATAAGAGACAG-3' |
| 2 | DNA Primer 19_2 | 5'-TCTACACATATTCTCTGTC-3' |
| 3 | carrier ssDNA | 5'-TCAGGTTTTCCTGAA-3' |
| 4 | LIANTI transposon DNA | 5'/Phos/CTGTCTCTTATACACATCTGAACAGAATTTAATACGACTCACTATAGGGAGATGTGTATAAGAGACAG-3' |
| 5 | DNA Primer 2nd strand | 5'-NNNNNNNNGGGAGATGTGTATAAGAGACAG-3' |

(continued)

| Seq. ID No. | Description | Sequence Information |
|---|---|---|
| 6 | DNA Primer 15_1 | 5'-AGATGTGTATAAGAG-3' |
| 7 | DNA Primer 25_1 | 5'-AGATGTGTATAAGAGACAGTCTACA-3' |
| 8 | DNA Primer 3+14N | 5'-NNNNNNNNNNNNNNGACAG-3' |
| 9 | DNA Primer 5+14N | 5'-AGA TGNNNNNNNNNNNNNN-3' |

**EMBODIMENTS**

Aspects of the present disclosure are directed to a method for amplifying the DNA in a solution, wherein the DNA content in the solution can be at the minimum of picogram level, the method including the following steps: combining lysis buffer with the solution, heating the solution, then combining protease with the solution, incubating the solution, followed by inactivation of the protease under high temperatures to obtain lysate; combining transposome with the lysate, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments; a 9-bp gap resulting from transposon insertion is filled and extended down to both ends of each fragment; performing in vitro transcription using the original sequence as a template to form RNA; combining the RNA with a DNA primer and a reverse transcription system to form a first strand cDNA via reverse transcription, wherein the DNA primer is complementary to the respective RNA strand of the sequence of transposition binding site; and forming a second strand DNA via cycling amplification. According to one aspect, the solution is a culture medium or a blastocoele fluid. According to one aspect, the DNA primer has a length of 15bp to 20bp. According to one aspect, the 3'-end of the DNA primer contains the sequence of 5'-GACAG-3 or 5'-CTGTC-3', preferably the DNA primer contains the sequence set force as 5'-GAT GTGTATAAGAGACAG-3' (Seq. ID No.: 1), or 5'-TCTACACATATTCTCTGTC-3' (Seq. ID No.: 2) or is at least 70%, preferably 80%, preferably 90%, preferably 95, preferably 100% identity with Seq. ID No.: 1 or Seq. ID No.: 2. According to one aspect, the lysis buffer is 2x to 10x lysis buffer. According to one aspect, the working concentration of the protease in the lysis mixture is higher than 0.6 mg/mL, preferably in the range of 0.8-2 mg/mL. According to one aspect, the incubation is performed at 45-60°C for 1 to 3 hours. According to one aspect, the inactivation is performed at 80-90°C. According to one aspect, the working concentration of the transposome is higher than 6 nM, preferably in the range of 10-40 nM. According to one aspect, the cycling amplification is performed more than 4 cycles, such as 4 to 20 cycles, preferably 6 to 15 cycles.

The present disclosure provides a method for amplifying the DNA in a culture medium, wherein the DNA content in the culture medium can be at the minimum of picogram level, the method includes the following steps: combining 2x to 10x lysis buffer with the culture medium, heating the culture medium, combining protease with the culture medium to the working concentration of 0.8-2 mg/mL and incubating the culture medium at 45-60°C for 1 to 3 hours, followed by inactivation of the protease at 80-90°C to obtain lysate; combining transposome with the lysate to the working concentration of 10-40 nM, wherein the transposome includes a transposase and a transposon, wherein the transposon comprises a transposase binding site and an RNA polymerase promoter sequence, wherein the transposome binds to DNA in the solution, the transposase cuts the DNA in the solution to produce DNA fragments and the transposon becomes incorporated or inserted into the DNA fragments; a 9-bp gap resulting from transposon insertion is filled and extended down to both ends of each fragment; performing in vitro transcription using the original sequence as a template to form RNA; combining the RNA with DNA primer pairing with the nucleic acid sequence conjugated with the transposome and a reverse transcription system, forming a first strand cDNA via reverse transcription, wherein the DNA primer contains the sequence set forth as 5'-AGATGTGTATAAGAGACAG-3' (Seq. ID No.: 1) or 5'-TCTACACATATTCTCTGTC-3' (Seq. ID No.: 2), or is at least 70%, preferably 80%, preferably 90%, preferably 95, preferably 100% identity with Seq. ID No.: 1 or Seq. ID No.: 2; and c. forming a second strand DNA via 6 to 15 cycles of amplification.

The present disclosure provides a method of analyzing a biological sample of culture medium of an in vitro cultured embryo to determine the susceptibility of a genetic disease in the embryo, including 1) amplifying the DNA molecules from the biological sample of culture medium of the in vitro cultured embryo and/or the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality (GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps: a) initially estimating the MCC rate and haplotype status of each SNP in the region adjacent to the disease-causing mutation site; and b) recursively updating the MCC rate and haplotype status of each SNP. According to one aspect, step b) includes using a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, after step 8), combining multiple identified SNPs of the embryo to determine its susceptibility to a polygenic disease.

The present disclosure provides a method of analyzing a biological sample of culture medium of an in vitro cultured embryo to determine the susceptibility of a genetic disease in the embryo, including 1) amplifying the DNA molecules from the biological sample of culture medium of the in vitro cultured embryo using the method according to any one of claims 1-12, and/or amplifying the DNA molecules from the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality (GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps a) initially estimating the MCC rate and haplotype status of each SNP in the region adjacent to the disease-causing mutation site; and b) recursively updating the MCC rate and haplotype status of each SNP. According to one aspect, step b) uses a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, step 8) includes combining multiple identified SNPs of the embryo to determine its susceptibility to a polygenic disease.

The present disclosure provides a computer program including a plurality of instructions capable of execution by a computer system and arranged when executed to control the computer system to analyze a biological sample of culture medium of an in vitro cultured embryo to determine the susceptibility of a genetic disease in the embryo, including one or more of the steps of 1) amplifying the DNA molecules from the biological sample of culture medium of the in vitro cultured embryo, and/or amplifying the DNA molecules from the biological sample of a discarded embryo in the family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by a computer system, for the blood samples of both parents or solely the disease-carrying parent; 5) calculating the likelihood of the four possible inheritance scenarios at each single SNP representing whether the genetic chains are inherited from the paternal or maternal alleles of the parents, taking into account the maternal cell contamination (MCC) rate and haplotype status; 6) estimating the MCC rate, and determining the haplotype status of each SNP to represent whether only DNA from one parent is present in the culture medium; 7) calculating the likelihood of observing the SNP data within regions adjacent to the disease-causing mutation site, combining the likelihood at each single SNP and the recombination probabilities; 8) determining whether the embryo carries the disease-causing chromosome, and providing a level of confidence. According to one aspect, the embryo has contamination caused by maternal cells and/or haplotype loss. According to one aspect, in step 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in step 3) controlling the quality of the SNP data is performed via filtering out the SNPs with low gene quality (GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in step 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in step 4) employing any one or more of the following samples in the family: the blood sample of proband, the biological sample of the discarded embryo, or the blood sample of at least one of the grandparents. According to one aspect, in step 5), integrating the estimated sequencing error rate when calculating the likelihood for each individual SNP. According to one aspect, step 6) further includes the following steps a) initially estimating the MCC rate and haplotype status of each SNP in the region adjacent to the disease-causing mutation site; and b) recursively updating the MCC rate and haplotype status of each SNP. According to one aspect, step b) uses a label-searching method. According to one aspect, after step a), excluding the sample with abnormal MCC rate. According to one aspect, the abnormal MCC rate is larger than 0.65 or less than -0.5. According to one aspect, in step 7) using a Bayesian model and a recursive algorithm. According to one aspect, in step 8), first plotting the log likelihood curve for both upstream and downstream regions of the disease-causing mutation site. According to one aspect, the genetic disease includes both monogenic and polygenic diseases. According to one aspect, after step 8), combining multiple identified SNPs of the embryo to determine its susceptibility to a polygenic disease.

The present disclosure provides a computer system for analyzing a biological sample of culture medium of an in vitro cultured embryo to determine the susceptibility of a genetic disease in the embryo, including 1) means for amplifying the DNA molecules from the biological sample of culture medium of the in vitro cultured embryo, and/or amplifying the biological sample of a discarded embryo in the family; 2) means for preparing DNA libraries and sequencing the DNA molecules from the biological sample to obtain a plurality of sequence reads; 3) means for performing mapping and SNP calling, by a computer system, to generate the SNP data and controlling its quality; 4) means for conducting haplotype pre-phasing, by a computer system, for the blood samples of both parents or solely the disease-carrying parent; 5) means for calculating the likelihood of the four possible inheritance scenarios at each single SNP representing whether the genetic chains are inherited from the paternal or maternal alleles of the parents, taking into account the maternal cell contamination (MCC) rate and haplotype status; 6) means for estimating the MCC rate, and determining the haplotype status of each SNP to represent whether only DNA from one parent is present in the culture medium; 7) means for calculating the likelihood of observing the SNP data within regions adjacent to the disease-causing mutation site, combining the likelihood at each single SNP and the recombination probabilities; 8) means for determining whether the embryo carries the disease-causing chromosome, and providing a level of confidence. According to one aspect, the embryo has contamination caused by maternal cells and/or haplotype loss. According to one aspect, in means 3) mapping and SNP calling outputs the physical location, allele type, allele depth and sequencing quality for each SNP of the biological sample of culture medium of the in vitro cultured embryo and/or the biological sample of the discarded embryo in the family. According to one aspect, in means 3) controlling the quality of the SNP data is performed via filtering out the SNPs with low gene quality (GQ) values of the biological sample of culture medium of the in vitro cultured embryo and/or the biological sample of the discarded embryo in the family. According to one aspect, including filtering out the SNPs with the lowest 20% GQ values of the biological sample of culture medium of the in vitro cultured embryo and/or the biological sample of the discarded embryo in the family. According to one aspect, in means 3), estimating the sequencing error rate and the relative density of SNPs in the region adjacent to the disease-causing mutation site, and excluding the sample of the culture medium with an extremely low SNP density, or high sequencing error rate. According to one aspect, the excluded SNP density is smaller than 0.001, or the excluded sequencing error rate is larger than 0.2. According to one aspect, in means 4) employing any one or more of the following samples in the family: the blood sample of proband, the biological sample of the discarded embryo, or the blood sample of at least one of the grandparents. According to one aspect, in means 5), integrating the estimated sequencing error rate when calculating the likelihood for each individual SNP. According to one aspect, means 6) further includes the following functions a) initially estimating the MCC rate and haplotype status of each SNP in the region adjacent to the disease-causing mutation site; and b) recursively updating the MCC rate and haplotype status of each SNP. According to one aspect, function b) uses a label-searching method. According to one aspect, after function a), excluding the sample with abnormal MCC rate. According to one aspect, the abnormal MCC rate is larger than 0.65 or less than -0.5. According to one aspect, in means 7) using a Bayesian model and a recursive algorithm. According to one aspect, in means 8), first plotting the log likelihood curve for both upstream and downstream regions of the disease-causing mutation site. According to one aspect, the genetic disease includes both monogenic and polygenic diseases. According to one aspect, further including means for combining multiple identified SNPs of the embryo to determine its susceptibility to a polygenic disease.

The present disclosure provides a method of preparing an otherwise unsequenceable biological sample of culture medium of an in vitro cultured embryo into a transformed state that is capable of being analyzed to determine a susceptibility of a genetic disease in the embryo, including 1) amplifying a DNA molecules from the biological sample of culture medium of the in vitro cultured embryo and/or the biological sample of a discarded embryo in a family; 2) preparing DNA libraries and sequencing the DNA molecules from the biological sample to obtain a plurality of sequence reads; 3) performing mapping and SNP calling, by a computer system, to generate the SNP data and controlling its quality; 4) conducting haplotype pre-phasing, by the computer system, for the blood samples of both parents or solely the disease-carrying parent; 5) calculating, by the computer system, the likelihood of the four possible inheritance scenarios at each single SNP representing whether the genetic chains are inherited from the paternal or maternal alleles of the parents, taking into account the maternal cell contamination (MCC) rate and haplotype status; 6) transforming the SNP data by estimating, by the computer system, the MCC rate, and determining the haplotype status of each SNP to represent whether only DNA from one parent is present in the culture medium; 7) calculating, by the computer system, the likelihood of observing the SNP data within regions adjacent to the disease-causing mutation site, combining the likelihood at each single SNP and the recombination probabilities; and 8) determining, by the computer system, whether the embryo carries the disease-causing chromosome, and providing a level of confidence, wherein the biological sample has high ADO rates in a presence of maternal contamination.

References

The following full citation references correspond respectively to the short form citations used above and are incorporated therein as if set forth fully therein.

1. Handyside AH, Penketh RJ, Winston RM, Pattinson JK, Delhanty JD, Tuddenham EG. Biopsy of human preimplantation embryos and sexing by DNA amplification. The Lancet. 1989 Feb 18;333(8634):347-9.
2. Handyside AH, Kontogianni EH, Hardy KR, Winston RM. Pregnancies from biopsied human preimplantation embryos sexed by Y-specific DNA amplification. Nature. 1990 Apr 19;344(6268):768-70.
3. Kerem BS, Rommens JM, Buchanan JA, Markiewicz D, Cox TK, Chakravarti A, Buchwald M, Tsui LC. Identification of the cystic fibrosis gene: genetic analysis. Science. 1989 Sep 8;245(4922):1073-80.
4. Handyside AH, Lesko JG, Tarin JJ, Winston RM, Hughes MR. Birth of a normal girl after in vitro fertilization and preimplantation diagnostic testing for cystic fibrosis. New England Journal of Medicine. 1992 Sep 24;327(13):905-9.
5. Liu J, Lissens W, Silber SJ, Devroey P, Liebaers I, Van Steirteghem A. Birth after preimplantation diagnosis of the cystic fibrosis∆ F508 mutation by polymerase chain reaction in human embryos resulting from intracytoplasmic sperm injection with epididymal sperm. Jama. 1994 Dec 21;272(23):1858-60.
6. Harton GL, Tsipouras P, Sisson ME, Starr KM, Mahoney BS, Fugger EF, Schulman JD, Kilpatrick MW, Levinson G, Black SH. Preimplantation genetic testing for Marfan syndrome. MHR: Basic science of reproductive medicine. 1996 Sep 1;2(9):713-5.

7. Ao A, Wells D, Handyside AH, Winston RM, Delhanty JD. Preimplantation genetic diagnosis of inherited cancer: familial adenomatous polyposis coli. Journal of assisted reproduction and genetics. 1998 Mar; 15:140-4.

8. Sermon K, Goossens V, Seneca S, Lissens W, De Vos A, Vandervorst M, Van Steirteghem A, Liebaers I. Preimplantation diagnosis for Huntington's disease (HD): clinical application and analysis of the HD expansion in affected embryos. Prenatal Diagnosis: Published in Affiliation With the International Society for Prenatal Diagnosis. 1998 Dec;18(13):1427-36.

9. Xu K, Shi ZM, Veeck LL, Hughes MR, Rosenwaks Z. First unaffected pregnancy using preimplantation genetic diagnosis for sickle cell anemia. Jama. 1999 May 12;281(18):1701-6.

10. Hussey ND, Donggui H, Froiland DA, Hussey DJ, Haan EA, Matthews CD, Craig JE. Analysis of five Duchenne muscular dystrophy exons and gender determination using conventional duplex polymerase chain reaction on single cells. Molecular human reproduction. 1999 Nov 1;5(11):1089-94.

11. Ray PF, Gigarel N, Paul Bonnefont J, Attié T, Hamamah S, Frydman N, Vekemans M, Frydman R, Munnich A. First specific preimplantation genetic diagnosis for ornithine transcarbamylase deficiency. Prenatal diagnosis. 2000 Dec;20(13):1048-54.

12. De Rycke M, Van de Velde H, Sermon K, Lissens W, De Vos A, Vandervorst M, Vanderfaeillie A, Van Steirteghem A, Liebaers I. Preimplantation genetic diagnosis for sickle-cell anemia and for β-thalassemia. Prenatal Diagnosis: Published in Affiliation With the International Society for Prenatal Diagnosis. 2001 Mar;21(3):214-22.

13. Moutou C, Gardes N, Rongieres C, Ohl J, Bettahar-Lebugle K, Wittemer C, Gerlinger P, Viville S. Allele-specific amplification for preimplantation genetic diagnosis (PGD) of spinal muscular atrophy. Prenatal diagnosis. 2001 Jun;21(6):498-503.

14. Verlinsky Y, Rechitsky S, Schoolcraft W, Strom C, Kuliev A. Preimplantation diagnosis for Fanconi anemia combined with HLA matching. Jama. 2001 Jun 27;285(24):3130-3.

15. Sermon K, Seneca S, De Rycke M, Goossens V, Van de Velde H, De Vos A, Platteau P, Lissens W, Van Steirteghem A, Liebaers I. PGD in the lab for triplet repeat diseases-myotonic dystrophy, Huntington's disease and Fragile-X syndrome. Molecular and Cellular Endocrinology. 2001 Oct 22; 183:S77-85.

16. Girardet A, Hamamah S, Anahory T, Dechaud H, Sarda P, Hedon B, Demaille J, Claustres M. First preimplantation genetic diagnosis of hereditary retinoblastoma using informative microsatellite markers. MHR: Basic science of reproductive medicine. 2003 Feb 1;9(2):111-6.

17. Fiorentino F, Biricik A, Nuccitelli A, De Palma R, Kahraman S, Iacobelli M, Trengia V, Caserta D, Bonu MA, Borini A, Baldi M. Strategies and clinical outcome of 250 cycles of preimplantation genetic diagnosis for single gene disorders. Human Reproduction. 2006 Mar 1;21(3):670-84.

18. Kahraman S, Beyazyurek C, Yesilipek MA, Ozturk G, Ertem M, Anak S, Kansoy S, Aksoylar S, Kuşkonmaz B, Oniz H, Slavin S. Successful haematopoietic stem cell transplantation in 44 children from healthy siblings conceived after preimplantation HLA matching. Reproductive biomedicine online. 2014 Sep 1;29(3):340-51.

19. Munné S, Lee A, Rosenwaks Z, Grifo J, Cohen J. Fertilization and early embryology: Diagnosis of major chromosome aneuploidies in human preimplantation embryos. Human reproduction. 1993 Dec 1;8(12):2185-91.

20. Wilton L, Williamson R, McBain J, Edgar D, Voullaire L. Birth of a healthy infant after preimplantation confirmation of euploidy by comparative genomic hybridization. New England Journal of Medicine. 2001 Nov 22;345(21):1537-41.

21. Wells D, Escudero T, Levy B, Hirschhorn K, Delhanty JD, Munne S. First clinical application of comparative genomic hybridization and polar body testing for preimplantation genetic diagnosis of aneuploidy. Fertility and sterility. 2002 Sep 1;78(3):543-9.

22. Treff NR, Su J, Tao X, Levy B, Scott Jr RT. Accurate single cell 24 chromosome aneuploidy screening using whole genome amplification and single nucleotide polymorphism microarrays. Fertility and sterility. 2010 Nov 1;94(6):2017-21.

23. Gutiérrez-Mateo C, Colls P, Sánchez-García J, Escudero T, Prates R, Ketterson K, Wells D, Munné S. Validation of microarray comparative genomic hybridization for comprehensive chromosome analysis of embryos. Fertility and sterility. 2011 Mar 1;95(3):953-8.

24. Yang Z, Liu J, Collins GS, Salem SA, Liu X, Lyle SS, Peck AC, Sills ES, Salem RD. Selection of single blastocysts for fresh transfer via standard morphology assessment alone and with array CGH for good prognosis IVF patients: results from a randomized pilot study. Molecular cytogenetics. 2012 Dec; 5:1-8.

25. Scott Jr RT, Upham KM, Forman EJ, Hong KH, Scott KL, Taylor D, Tao X, Treff NR. Blastocyst biopsy with comprehensive chromosome screening and fresh embryo transfer significantly increases in vitro fertilization implantation and delivery rates: a randomized controlled trial. Fertility and sterility. 2013 Sep 1;100(3):697-703.

26. Forman EJ, Hong KH, Ferry KM, Tao X, Taylor D, Levy B, Treff NR, Scott Jr RT. In vitro fertilization with single euploid blastocyst transfer: a randomized controlled trial. Fertility and sterility. 2013 Jul 1;100(1):100-7.

27. Wells D, Kaur K, Grifo J, Glassner M, Taylor JC, Fragouli E, Munne S. Clinical utilisation of a rapid low-pass whole genome sequencing technique for the diagnosis of aneuploidy in human embryos prior to implantation. Journal of medical genetics. 2014 Aug 1;51(8):553-62.

28. Rubio C, Bellver J, Rodrigo L, Castillón G, Guillén A, Vidal C, Giles J, Ferrando M, Cabanillas S, Remohi J, Pellicer A. In vitro fertilization with preimplantation genetic diagnosis for aneuploidies in advanced maternal age: a randomized, controlled study. Fertility and sterility. 2017 May 1;107(5):1122-9.

29. Conn CM, Harper JC, Winston RM, Delhanty JD. Infertile couples with Robertsonian translocations: preimplantation genetic analysis of embryos reveals chaotic cleavage divisions. Human Genetics. 1998 Jan; 102:117-23.

30. Scriven PN, Handyside AH, Ogilvie CM. Chromosome translocations: segregation modes and strategies for preimplantation genetic diagnosis. Prenatal Diagnosis: Published in Affiliation With the International Society for Prenatal Diagnosis. 1998 Dec;18(13):1437-49.

31. Coonen E, Martini E, Dumoulin JC, Hollanders-Crombach HT, de Die-Smulders C, Geraedts JP, Hopman AH, Evers JL. Preimplantation genetic diagnosis of a reciprocal translocation t (3; 11)(q27. 3; q24. 3) in siblings. Molecular human reproduction. 2000 Mar 1;6(3):199-206.

32. Munné S, Sandalinas M, Escudero T, Fung J, Gianaroli L, Cohen J. Outcome of preimplantation genetic diagnosis of translocations. Fertility and Sterility. 2000 Jun 1;73(6):1209-18.

33. Escudero T, Abdelhadi I, Sandalinas M, Munne S. Predictive value of sperm fluorescence in situ hybridization analysis on the outcome of preimplantation genetic diagnosis for translocations. Fertility and sterility. 2003 Jun 1; 79:1528-34.

34. Melotte C, Debrock S, D'Hooghe T, Fryns JP, Vermeesch JR. Preimplantation genetic diagnosis for an insertional translocation carrier. Human Reproduction. 2004 Dec 1;19(12):2777-83.

35. Le Caignec C, Spits C, Sermon K, De Rycke M, Thienpont B, Debrock S, Staessen C, Moreau Y, Fryns JP, Van Steirteghem A, Liebaers I. Single-cell chromosomal imbalances detection by array CGH. Nucleic acids research. 2006 Jan 1;34(9):e68-.

36. Traversa MV, Carey L, Leigh D. A molecular strategy for routine preimplantation genetic diagnosis in both reciprocal and Robertsonian translocation carriers. MHR: Basic science of reproductive medicine. 2010 Feb 19;16(5):329-37.

37. Fiorentino F, Kokkali G, Biricik A, Stavrou D, Ismailoglu B, De Palma R, Arizzi L, Harton G, Sessa M, Pantos K. Polymerase chain reaction-based detection of chromosomal imbalances on embryos: the evolution of preimplantation genetic diagnosis for chromosomal translocations. Fertility and sterility. 2010 Nov 1;94(6):2001-11.

38. Rius M, Obradors A, Daina G, Ramos L, Pujol A, Martinez-Passarell O, Marquès L, Oliver-Bonet M, Benet J, Navarro J. Detection of unbalanced chromosome segregations in preimplantation genetic diagnosis of translocations by short comparative genomic hibridization. Fertility and sterility. 2011 Jul 1;96(1):134-42.

39. Fiorentino F, Spizzichino L, Bono S, Biricik A, Kokkali G, Rienzi L, Ubaldi FM, Iammarrone E, Gordon A, Pantos K. PGD for reciprocal and Robertsonian translocations using array comparative genomic hybridization. Human Reproduction. 2011 Jul 1;26(7):1925-35.

40. Treff NR, Thompson K, Rafizadeh M, Chow M, Morrison L, Tao X, Garnsey H, Reda CV, Metzgar TL, Neal S, Jalas C. SNP array-based analyses of unbalanced embryos as a reference to distinguish between balanced translocation carrier and normal blastocysts. Journal of Assisted Reproduction and Genetics. 2016 Aug; 33:1115-9.

41. Zhang S, Lei C, Wu J, Zhou J, Sun H, Fu J, Sun Y, Sun X, Lu D, Zhang Y. The establishment and application of preimplantation genetic haplotyping in embryo diagnosis for reciprocal and Robertsonian translocation carriers. BMC medical genomics. 2017 Dec;10(1):1-9.

42. Tan YQ, Tan K, Zhang SP, Gong F, Cheng DH, Xiong B, Lu CF, Tang XC, Luo KL, Lin G, Lu GX. Single-nucleotide polymorphism microarray-based preimplantation genetic diagnosis is likely to improve the clinical outcome for translocation carriers. Human Reproduction. 2013 Sep 1;28(9):2581-92.

43. Chow JF, Yeung WS, Lee VC, Lau EY, Ng EH. Evaluation of preimplantation genetic testing for chromosomal structural rearrangement by a commonly used next generation sequencing workflow. European Journal of Obstetrics & Gynecology and Reproductive Biology. 2018 May 1; 224:66-73.

44. Treff NR, Eccles J, Lello L, Bechor E, Hsu J, Plunkett K, Zimmerman R, Rana B, Samoilenko A, Hsu S, Tellier LC. Utility and first clinical application of screening embryos for polygenic disease risk reduction. Frontiers in endocrinology. 2019 Dec 4; 10:845.

45. Kumar A, Im K, Banjevic M, Ng PC, Tunstall T, Garcia G, Galhardo L, Sun J, Schaedel ON, Levy B, Hongo D. Whole-genome risk prediction of common diseases in human preimplantation embryos. Nature medicine. 2022 Mar;28(3):513-6.

46. Yan L, Huang L, Xu L, Huang J, Ma F, Zhu X, et al. Live births after simultaneous avoidance of monogenic diseases and chromosome abnormality by next-generation sequencing with linkage analyses. Proc Natl Acad Sci U S A 2015; 112:15964-9

47. Dokras A, Sargent IL, Ross C, Gardner RL, Barlow DH. Trophectoderm biopsy in human blastocysts. Hum Reprod 1990;5: 821-825.

48. Kokkali G, Vrettou C, Traeger-Synodinos J, Jones GM, Cram DS, Stavrou D, Trounson AO, Kanavakis E, Pantos K. Birth of a healthy infant following trophectoderm biopsy from blastocysts for PGD of β-thalassaemia major: case report. Hum Reprod 2005; 20:1855-1859.

49. Palini S, Galluzzi L, De Stefani S, Bianchi M,Wells D, Magnani M, Bulletti C. Genomic DNA in human blastocoele fluid. Reprod Biomed Online 2013; 26:603-610.

50. Gianaroli L, Magli MC, Pomante A, Crivello AM, Cafueri G, Valerio M, Ferraretti AP. Blastocentesis: a source of DNA for preimplantation genetic testing. Results from a pilot study. Fertil Steril 2014; 102:1692-1699.

51. Tobler KJ, Zhao Y, Ross R, Benner AT, Xu X, Du L, Broman K, Thrift K, Brezina PR, Kearns WG. Blastocoel fluid from differentiated blastocysts harbors embryonic genomic material capable of a whole-genome deoxyribonucleic acid amplification and comprehensive chromosome microarray analysis. Fertil Steril 2015;104: 418-425.

52. Magli MC, Pomante A, Cafueri G, Valerio M, Crippa A, Ferraretti AP, Gianaroli L. Preimplantation genetic testing: Polar bodies, blastomeres, trophectoderm cells, or blastocoelic fluid? Fertil Steril 2016; 105:676-683e5.

53. Zhang Y, Li N, Wang L, Sun H, Ma M, Wang H, Xu X, Zhang W, Liu Y, Cram DS et al. Molecular analysis of DNA in blastocoele fluid using next-generation sequencing. J Assist Reprod Genet 2016;33: 637-645.

54. Shangguan T, He W, Li H, Shang X, Liu Y, Bai X, Li M, Xie J. Detection and analysis of DNA material in human blastocoel fluid. Biomed Genet Genomics 2017; 2:1-5.

55. Capalbo A, Romanelli V, Patassini C, Poli M, Girardi L, Giancani A, Stoppa M, Cimadomo D, Ubaldi FM, Rienzi L. Diagnostic efficacy of blastocoel fluid and spent media as sources of DNA for preimplantation genetic testing in standard clinical conditions. Fertil Steril 2018; 110:870-879.

56. Tšuiko O, Zhigalina DI, Jatsenko T, Skryabin NA, Kanbekova OR, Artyukhova VG, Svetlakov AV, Teearu K, Trošin A, Salumets A, Kurg A. Karyotype of the blastocoel fluid demonstrates low concordance with both trophectoderm and inner cell mass. Fertility and sterility. 2018 Jun 1;109(6):1127-34.

57. Magli MC, Albanese C, Crippa A, Tabanelli C, Ferraretti AP, Gianaroli L. Deoxyribonucleic acid detection in blastocoelic fluid: a new predictor of embryo ploidy and viable pregnancy. Fertil Steril 2018; 111:77-85.

58. Galluzzi L, Palini S, De Stefani S, Andreoni F, Primiterra M, Diotallevi A, Bulletti C, Magnani M. Extracellular embryo genomic DNA and its potential for genotyping applications. Futur Sci OA 2015;1: FSO62.

59. Wu H, Ding C, Shen X, Wang J, Li R, Cai B, Xu Y, Zhong Y, Zhou C. Medium-based noninvasive preimplantation genetic diagnosis for human α-thalassemias-SEA. Medicine (Baltimore) 2015;94: e669.

60. Xu J, Fang R, Chen L, Chen D, Xiao J-P, Yang W, Wang H, Song X, Ma T, Bo S et al. Noninvasive chromosome screening of human embryos by genome sequencing of embryo culture medium for in vitro fertilization. Proc Natl Acad Sci 2016; 113:11907-11912.

61. Shamonki MI, Jin H, Haimowitz Z, Liu L. Proof of concept: preimplantation genetic screening without embryo biopsy through analysis of cell-free DNA in spent embryo culture media. Fertil Steril 2016; 106:1312-1318.

62. Feichtinger M, Vaccari E, Carli L, Wallner E, Mädel U, Figl K, Palini S, Feichtinger W. Non-invasive preimplantation genetic screening using array comparative genomic hybridization on spent culture media: a proof-of-concept pilot study. Reprod Biomed Online 2017; 34:583-589.

63. Lane M, Zander-Fox DL, Hamilton H, Jasper MJ, Hodgson BL, Fraser M, Bell F. Ability to detect aneuploidy from cell free DNA collected from media is dependent on the stage of development of the embryo. Fertil Steril 2017; 108:e61.

64. Liu WQ, Liu JQ, Du HZ, Ling JW, Sun XF, Chen DJ. Non-invasive preimplantation aneuploidy screening and diagnosis of beta thalassemia IVSII654 mutation using spent embryo culture medium. Ann Med 2017; 49:319-328.

65. Ho JR, Arrach N, Rhodes-Long K, Ahmady A, Ingles S, Chung K, Bendikson KA, Paulson RJ, McGinnis LK. Pushing the limits of detection: investigation of cell-free DNA for aneuploidy screening in embryos. Fertil Steril 2018; 110:467-475.e2.

66. Vera-Rodriguez M, Diez-Juan A, Jimenez-Almazan J, Martinez S, Navarro R, Peinado V, Mercader A, Meseguer M, Blesa D, Moreno I et al. Origin and composition of cell-free DNA in spent medium from human embryo culture during preimplantation development. Obstet Gynecol Surv 2018; 33:745-756.

67. Capalbo A, Romanelli V, Patassini C, Poli M, Girardi L, Giancani A, Stoppa M, Cimadomo D, Ubaldi FM, Rienzi L. Diagnostic efficacy of blastocoel fluid and spent media as sources of DNA for preimplantation genetic testing in standard clinical conditions. Fertil Steril 2018; 110:870-879.

68. Fang R, Yang W, Zhao X, Xiong F, Guo C, Xiao J, Chen L, Song X, Wang H, Chen J et al. Chromosome screening using culture medium of embryos fertilised in vitro: A pilot clinical study. J Transl Med 2019; 17:1-8.

69. Huang L, Bogale B, Tang Y, Lu S, Xie XS, Racowsky C. Noninvasive preimplantation genetic testing for aneuploidy in spent medium may be more reliable than trophectoderm biopsy. Proc Natl Acad Sci 2019; 116:201907472

70. Rubio C, Rienzi L, Navarro-Sánchez L, Cimadomo D, Garcia-Pascual CM, Albricci L, Soscia D, Valbuena D, Capalbo A, Ubaldi F et al. Embryonic cell-free DNA versus trophectoderm biopsy for aneuploidy testing: concordance rate and clinical implications. Fertil Steril 2019; 112:510-519.

71. Yeung QSY, Zhang YX, Chung JPW, Lui WT, Kwok YKY, Gui B, Kong GWS, Cao Y, Li TC, Choy KW. A prospective study of non-invasive preimplantation genetic testing for aneuploidies (NiPGT-A) using next-generation sequencing (NGS) on spent culture media (SCM). J Assist Reprod Genet 2019; 36:1609-1621.

72. Jiao J, Shi B, Sagnelli M, Yang D, Yao Y, LiW, Shao L, Lu S, Li D,Wang X. Minimally invasive preimplantation genetic testing using blastocyst culture medium. Hum Reprod 2019; 34:1369-1379.

73. Ou Z, Deng Y, Liang Y, Chen Z, Sun L. Improved non-invasive preimplantation genetic testing for beta-thalassemia using spent embryo culture medium containing blastocoelic fluid. Frontiers in endocrinology. 2022 Jan 20; 12:1941.

74. Galluzzi L, Palini S, De Stefani S, Andreoni F, Primiterra M, Diotallevi A, Bulletti C, Magnani M. Extracellular embryo genomic DNA and its potential for genotyping applications. Futur Sci OA 2015;1: FSO62.

75. Chan KA, Zhang J, Hui AB, Wong N, Lau TK, Leung TN, Lo KW, Huang DW, Lo YD. Size distributions of maternal and fetal DNA in maternal plasma. Clinical chemistry. 2004 Jan 1;50(1):88-92.

76. Huang L, Ma F, Chapman A, Lu S, Xie XS. Single-cell whole-genome amplification and sequencing: methodology and applications. Annual review of genomics and human genetics. 2015 Aug 24; 16:79-102.

77. Chen C, Xing D, Tan L, Li H, Zhou G, Huang L, Xie XS. Single-cell whole-genome analyses by Linear Amplification via Transposon Insertion (LIANTI). Science. 2017 Apr 14;356(6334):189-94.

78. CHEN, Chongyi; XING, Dong; XIE, Xiaoliang Sunney. Methods of amplifying nucleic acid sequences mediated by transposase/transposon DNA complexes. U.S. Patent No 10,894,980, 2021.

79. Leaver M, Wells D. Non-invasive preimplantation genetic testing (niPGT): the next revolution in reproductive genetics?. Human reproduction update. 2020 Jan 1;26(1):16-42.

80. Cimadomo D, Rienzi L, Capalbo A, Rubio C, Innocenti F, Garcia-Pascual CM, Ubaldi FM, Handyside A. The dawn of the future: 30 years from the first biopsy of a human embryo. The detailed history of an ongoing revolution. Human Reproduction Update. 2020 Jul;26(4):453-73.

81. De Rycke M., Goossens V., Kokkali G., Meijer-Hoogeveen M., Coonen E., Moutou C. ESHRE PGD Consortium data collection XIV-XV: Cycles from January 2011 to December 2012 with pregnancy follow-up to October 2013. Hum. Reprod. 2017; 32:1974-1994. doi: 10.1093/humrep/dex265.

82. ESHRE PGT-M Working Group, Carvalho F, Moutou C, Dimitriadou E, Dreesen J, Giménez C, Goossens V, Kakourou G, Vermeulen N, Zuccarello D, De Rycke M. ESHRE PGT Consortium good practice recommendations for the detection of monogenic disorders. Human reproduction open. 2020(3):1-18. Doi:10.1093/hropen/hoaa018.

83. Xue, A. et al. Genome-wide association analyses identify 143 risk variants and putative regulatory mechanisms for type 2 diabetes. Nat. Commun. 9, 2941 (2018).

84. Fan, H. C., Gu, W., Wang, J., Blumenfeld, Y. J., El-Sayed, Y. Y., & Quake, S. R. (2012). Non-invasive prenatal measurement of the fetal genome. Nature, 487(7407), 320-324.

85. Nabieva, E, Sharma, S M, Kapushev Y, et al. Accurate fetal variant calling in the presence of maternal cell contamination. European Journal of Human Genetics, 2020, 28(11): 1615-1623.

86. Lander, E.S. and Green, P. Construction of multilocus genetic linkage maps in humans, Proceedings of the National Academy of Sciences, 1987; 84 (8), 2363-2367

87. Kruglyak L, Daly M J, Reeve-Daly M P, et al. Parametric and nonparametric linkage analysis: a unified multipoint approach. American journal of human genetics, 1996, 58(6): 1347.

88. Idury R M, Elston R C. A faster and more general hidden Markov model algorithm for multipoint likelihood calculations. Human heredity, 1997, 47(4): 197-202.

89. Kruglyak L, Lander E S. Faster multipoint linkage analysis using Fourier transforms. J. Comput. Biol. 1998;5(1):1-7.

90. Abecasis G R, Cherny S S, Cookson W O, et al. Merlin-rapid analysis of dense genetic maps using sparse gene flow trees. Nature genetics, 2002, 30(1): 97-101.

**Claims**

1. A method of analyzing a biological sample of culture medium of an in vitro cultured embryo to determine the susceptibility of a genetic disease in the embryo, comprising:

   1) Amplifying the DNA molecules from the biological sample of culture medium of the in vitro cultured embryo;
   2) Preparing DNA libraries and sequencing the DNA molecules from the biological sample to obtain a plurality of sequence reads;
   3) Performing mapping and SNP calling on the plurality of sequence reads, by a computer system, to generate the SNP data and controlling its quality;
   4) Conducting haplotype pre-phasing, by a computer system, for the blood samples of both parents or solely the disease-carrying parent;
   5) Calculating the likelihood of the four possible inheritance scenarios at each single SNP representing whether the genetic chains are inherited from the paternal or maternal alleles of the parents, taking into account the maternal cell contamination (MCC) rate and haplotype status;

6) Estimating the MCC rate, and determining the haplotype status of each SNP to represent whether only DNA from one parent is present in the culture medium;

7) Calculating the likelihood of observing the SNP data within regions adjacent to the disease-causing mutation site, combining the likelihood at each single SNP and the recombination probabilities;

8) Determining whether the embryo carries the disease-causing chromosome, and providing a level of confidence;

wherein the MCC rate is defined as the proportion of maternal DNA in the culture medium of the embryo compared to the total amount of DNA.

2. The method of claim 1, wherein the embryo has contamination caused by maternal cells and/or haplotype loss.

3. The method of claim 1 or 2, wherein

(i) in step 3), mapping and SNP calling outputs the physical location, allele type, allele depth and sequencing quality for each SNP of the biological sample of culture medium of the in vitro cultured embryo;

(ii) in step 3), controlling the quality of the SNP data is performed via filtering out the SNPs with low gene quality (GQ) values, preferably with the lowest 20% GQ values, of the biological sample of culture medium of the in vitro cultured embryo;

(iii) in step 3), estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;

(iv) in step 4), employing any one or more of the following samples in the family: the blood sample of proband, or the blood sample of at least one of the grandparents;

(v) in step 5), integrating the estimated sequencing error rate when calculating the likelihood for each individual SNP;

(vi) step 6) further includes the following steps:

a) Initially estimating the MCC rate and haplotype status of each SNP in the region adjacent to the disease-causing mutation site; and

b) Recursively updating the MCC rate and haplotype status of each SNP, preferably using a label-searching method,

wherein after step a), preferably excluding the samples with abnormal MCC rate, wherein preferably the abnormal MCC rate is larger than 0.65 or less than -0.5;

(vii) in step 7), using a Bayesian model and a recursive algorithm;

(viii) in step 8), first plotting the log likelihood curve for both upstream and downstream regions of the disease-causing mutation site, or

(ix) after step 8), the method further comprises combining multiple identified SNPs of the embryo to determine its susceptibility to a polygenic disease.

4. The method of any one of claims 1 to 3, wherein the genetic disease includes both monogenic and polygenic diseases.

5. The method of any one of claims 1 to 4, wherein Step 1) is performed by an amplification process comprising the following steps:

A1) combining a lysis buffer with the biological sample to obtain a solution, heating the solution, then combining a protease with the solution, incubating the solution, followed by inactivation of the protease under a high temperature to obtain lysate;

A2) combining transposome with the lysate, wherein the transposome includes a transposase and a transposon, wherein the transposon comprises a transposase binding site at its 5' end, a transposase binding site at its 3' end, and an RNA polymerase promoter sequence between the two transposase binding sites, wherein the transposome binds to the DNA in the solution, the transposase cuts the DNA in the solution to produce DNA fragments and the transposon becomes incorporated or inserted into the DNA fragments;

A3) filling a gap resulting from the transposon or insertion and extending the DNA fragment down to both ends of each fragment;

A4) performing in vitro transcription with an RNA polymerase using the original sequence of the DNA fragment as a template to form an RNA comprising a 3'-end sequence complementary to the complete transposon;

A5) combining the RNA with a DNA primer and a reverse transcription system to form a first strand cDNA via reverse transcription, wherein the DNA primer is complementary to a region in the 3'-end sequence of the RNA that is complementary to one of the two transposase binding sites in the transposon; and

A6) forming a second strand DNA via cycling amplification.

6. The method of claim 5, wherein the amplification process is performed under one or more conditions selected from the group consisting of the following (i) to (xii):

(i) the lysis buffer is 2x to 10x lysis buffer,

(ii) the working concentration of the protease in the lysis mixture in step A1) is higher than 0.6 mg/mL, or is in the range of 0.8-2 mg/mL,

(iii) the incubating in step A1) is performed at 45-60°C for 1 to 3 hours,

(iv) the inactivation in step A1) is performed at 80-90°C,

(v) the working concentration of the transposome in step A2) is higher than 6 nM, or is in the range of 10-40 nM,

(vi) the transposase in the transposome in step A2) is a Tn5 transposase, and the transposase binding site in the transposon is a 19 bp Tn5 transposase binding site,

(vii) the transposon in the transposome in step A2) has a sequence of Seq. ID No.: 4,

(viii) the gap resulting from the transposon incorporation or insertion in step A3) is a 9-bp gap,

(ix) the filling and extending in step A3) are performed in the presence of a DNA polymerase which is inactivated before step A4),

(x) the RNA polymerase in step A4) is a T7 RNA polymerase,

(xi) the DNA primer in step A5) has a length of 15bp to 20bp, and

(xii) the cycling amplification in step A6) is performed more than 4 cycles, or 4 to 20 cycles, or 6 to 15 cycles.

7. The method of claim 5, wherein the DNA primer in step A5) has a length of 15bp to 20bp, and the 3'-end of the DNA primer contains the sequence of 5'-GACAG-3' or 5'-CTGTC-3',

preferably wherein the DNA primer contains the sequence of 5'-AGATGTGTATAAGAGACAG-3' (Seq. ID No.: 1) or 5'-TCTACACATATTCTCTGTC-3' (Seq. ID No.: 2) or has at least 70%, at least 80%, at least 90%, at least 95%, or 100% identity with Seq. ID No.: 1 or Seq. ID No.: 2.

8. The method of claim 5, wherein

in step A1), the working concentration of the protease in the lysis mixture is 0.8-2 mg/mL, the incubating is performed at 45-60°C for 1 to 3 hours, and the inactivation is performed at 80-90°C,

in step A2), the working concentration of the transposome is 10-40 nM,

in step A3), the gap resulting from the transposon incorporation or insertion is a 9-bp gap,

in step A5), the DNA primer contains the sequence of 5'-AGATGTGTATAAGAGACAG-3' (Seq. ID No.: 1) or 5'-TCTACACATATTCTCTGTC-3' (Seq. ID No.: 2) or has at least 70%, at least 80%, at least 90%, at least 95%, or 100% identity with Seq. ID No.: 1 or Seq. ID No.: 2, and in step A6), the cycling amplification is performed 6 to 15 cycles.

9. A computer program comprising a plurality of instructions capable of being executed by a computer system and arranged when executed to control the computer system to analyze a biological sample of culture medium of an in vitro cultured embryo to determine the susceptibility of a genetic disease in the embryo, wherein the computer program is configured, when executed, to cause the computer system to perform Steps 3) to 8) of the method of any one of claims 1 to 8, wherein Step 3) of the method is performed on the plurality of sequence reads obtained after Steps 1) and 2) of the method.

10. The program of claim 9, wherein the computer program is configured, when executed, to cause the computer system to perform, after step 8) of the method, combining multiple identified SNPs of the embryo to determine its susceptibility to a polygenic disease.

11. Computer system for analyzing a biological sample of culture medium of an in vitro cultured embryo to determine the susceptibility of a genetic disease in the embryo, comprising

Means 1) to 6) for performing Steps 3) to 8) of the method of any one of claims 1-8, respectively, wherein Means 1) is for performing Step 3) of the method on the plurality of sequence reads obtained after Steps 1) and 2) of the method; and

optionally Means 7) for combining multiple identified SNPs of the embryo to determine its susceptibility to a polygenic disease.

12. A method of preparing an otherwise unsequenceable biological sample of culture medium of an in vitro cultured embryo into a transformed state that is capable of being analyzed to determine a susceptibility of a genetic disease in the embryo, comprising:

1) amplifying a DNA molecules from the biological sample of culture medium of the in vitro cultured embryo;

2) preparing DNA libraries and sequencing the DNA molecules from the biological sample to obtain a plurality of sequence reads;

3) performing mapping and SNP calling on the plurality of sequence reads, by a computer system, to generate the SNP data and controlling its quality;

4) conducting haplotype pre-phasing, by the computer system, for the blood samples of both parents or solely the disease-carrying parent;

5) calculating, by the computer system, the likelihood of the four possible inheritance scenarios at each single SNP representing whether the genetic chains are inherited from the paternal or maternal alleles of the parents, taking into account the maternal cell contamination (MCC) rate and haplotype status;

6) transforming the SNP data by estimating, by the computer system, the MCC rate, and determining the haplotype status of each SNP to represent whether only DNA from one parent is present in the culture medium;

7) calculating, by the computer system, the likelihood of observing the SNP data within regions adjacent to the disease-causing mutation site, combining the likelihood at each single SNP and the recombination probabilities; and

8) determining, by the computer system, whether the embryo carries the disease-causing chromosome, and providing a level of confidence,

wherein the biological sample has a high Allele Drop-Out (ADO) rate, preferably higher than 5%, in a presence of maternal contamination;

wherein the MCC rate is defined as the proportion of maternal DNA in the culture medium of the embryo compared to the total amount of DNA.

13. The method of claim 12, wherein

(i) in step 3), mapping and SNP calling outputs the physical location, allele type, allele depth and sequencing quality for each SNP of the biological sample of culture medium of the in vitro cultured embryo;

(ii) in step 3), controlling the quality of the SNP data is performed via filtering out the SNPs with low gene quality (GQ) values, preferably with the lowest 20% GQ values, of the biological sample of culture medium of the in vitro cultured embryo;

(iii) in step 3), estimating the sequencing error rate and the relative density of SNPs in the region adjacent to the disease-causing mutation site, and excluding the sample of the culture medium with an extremely low SNP density, or high sequencing error rate, wherein preferably the excluded SNP density is smaller than 0.001, or the excluded sequencing error rate is larger than 0.2;

(iv) in step 4), employing any one or more of the following samples in the family: the blood sample of proband, or the blood sample of at least one of the grandparents;

(v) in step 5), integrating the estimated sequencing error rate when calculating the likelihood for each individual SNP;

(vi) step 6) further includes the following steps:

a) Initially estimating the MCC rate and haplotype status of each SNP in the region adjacent to the disease-causing mutation site; and

b) Recursively updating the MCC rate and haplotype status of each SNP, preferably using a label-searching method,

wherein after step a), preferably excluding the samples with abnormal MCC rate, wherein preferably the abnormal MCC rate is larger than 0.65 or less than -0.5;

(vii) in step 7), using a Bayesian model and a recursive algorithm;

(viii) in step 8), first plotting the log likelihood curve for both upstream and downstream regions of the disease-causing mutation site,

(ix) after step 8), the method further comprises combining multiple identified SNPs of the embryo to determine its susceptibility to a polygenic disease,

(x) the embryo has contamination caused by maternal cells and/or haplotype loss, or

(xi) the genetic disease includes both monogenic and polygenic diseases.

14. The method of claim 12 or 13, wherein Step 1) is performed by the amplification process defined in any one of claims 5 to 8.

Amplifying the DNA molecules from the biological sample of culture medium of the in vitro cultured embryo

Conducting haplotype pre-phasing, by a computer system, for the blood samples of both parents or solely the disease-carrying parent

Sequencing and performing mapping and SNP calling to the DNA molecules from the biological sample of culture medium of the in vitro cultured embryo

Calculating the likelihood at each single SNP, and recursively estimating the MCC rate and determining the haplotype status of each SNP

Calculating the likelihood of observing the SNP data within regions adjacent to the disease-causing mutation site

Determining whether the embryo carries the disease-causing chromosome, and providing a level of confidence

Fig. 1

Fig. 2

Fig. 3

Whole Genome Coverage of Case 1 samples

Fig. 4

Fig. 5

Fig. 6

Fig. 7

(a)

1-SCM-02 niPGT-A

1-TE-02 niPGT-A: 45, XN, -10(x1)

(b)

1-SCM-04 niPGT-A

1-TE-04 niPGT-A: 46, XN, -9q(x1,mos,~43%)

(c)

1-SCM-14 niPGT-A

1-TE-14 niPGT-A: 46,XN

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10894980 B
- WO 9810077 A
- EP 2527438 A
- EP 2376517 A

**Non-patent literature cited in the description**

- **MAEKAWA, T** ; **YANAGIHARA, K** ; **OHTSUBO, E.** A cell-free system of Tn3 transposition and transposition immunity. *Genes Cells*, 1996, vol. 1, 1007-1016
- **CRAIG, N.L**. Tn7: a target site-specific transposon. *Mol. Microbiol.*, 1991, vol. 5, 2569-2573
- **CHALMERS, R.** ; **SEWITZ, S.** ; **LIPKOW, K.** ; **CRELLIN, P**. Complete nucleotide sequence of Tn10. *J. Bacteriol*, 2000, vol. 182, 2970-2972
- **LI, X.** ; **BURNIGHT, E.R.** ; **COONEY, A.L** ; **MALANI, N.** ; **BRADY, T** ; **SANDER, J.D** ; **STABER, J.** ; **WHEELAN, S.J.** ; **JOUNG, J.K** ; **MCCRAY, P.B., JR. et al.** PiggyBac transposase tools for genome engineering. *Proc. Natl. Acad. Sci. USA*, 2013, vol. 110, E2279-2287
- **IVICS, Z.** ; **HACKETT, P.B.** ; **PLASTERK, R.H** ; **IZSVAK, Z**. Molecular reconstruction of Sleeping Beauty, a Tc1-like transposon from fish, and its transposition in human cells. *Cell*, 1997, vol. 91, 501-510
- **KAWAKAMI, K.** Tol2: a versatile gene transfer vector in vertebrates. *Genome Biol.*, 2007, vol. 8 (1), S7
- **JORGENSEN, E.D.** ; **DURBIN, R.K** ; **RISMAN, S.S** ; **MCALLISTER, W.T.** Specific contacts between the bacteriophage T3, T7, and SP6 RNA polymerases and their promoters. *J. Biol. Chem.*, 1991, vol. 266, 645-651
- **MELTON, D.A.** ; **KRIEG, P.A** ; **REBAGLIATI, M.R** ; **MANIATIS, T** ; **ZINN, K.** ; **GREEN, M.R**. Efficient in vitro synthesis of biologically active RNA and RNA hybridization probes from plasmids containing a bacteriophage SP6 promoter. *Nucleic Acids Res.*, 1984, vol. 12, 7035-7056
- **GORYSHIN, I.Y.** ; **W.S. REZNIKOFF**. Tn5 in vitro transposition. *The Journal of biological chemistry*, 1998, vol. 273 (13), 7367-74
- **DAVIES, D.R. et al.** Three-dimensional structure of the Tn5 synaptic complex transposition intermediate. *Science*, 2000, vol. 289 (5476), 77-85
- **GORYSHIN, I.Y. et al.** Insertional transposon mutagenesis by electroporation of released Tn5 transposition complexes. *Nature biotechnology*, 2000, vol. 18 (1), 97-100
- **STEINIGER-WHITE, M** ; **I. RAYMENT** ; **W.S. REZNIKOFF**. Structure/function insights into Tn5 transposition. *Current opinion in structural biology*, 2004, vol. 14 (1), 50-7
- **ADEY, A. et al.** Rapid, low-input, low-bias construction of shotgun fragment libraries by high-density in vitro transposition. *Genome biology*, 2010, vol. 11 (12), R119
- **MARINE, R. et al.** Evaluation of a transposase protocol for rapid generation of shotgun high-throughput sequencing libraries from nanogram quantities of DNA. *Applied and environmental microbiology*, 2011, vol. 77 (22), 8071-9
- **PARKINSON, N.J. et al.** Preparation of high-quality next-generation sequencing libraries from picogram quantities of target DNA. *Genome research*, 2012, vol. 22 (1), 125-33
- **ADEY, A** ; **J. SHENDURE**. Ultra-low-input, tagmentation-based whole-genome bisulfite sequencing. *Genome research*, 2012, vol. 22 (6), 1139-43
- **PICELLI, S. et al.** Full-length RNA-seq from single cells using Smart-seq2. *Nature protocols*, 2014, vol. 9 (1), 171-81
- **BUENROSTRO, J.D. et al.** Transposition of native chromatin for fast and sensitive epigenomic profiling of open chromatin, DNA-binding proteins and nucleosome position. *Nature methods*, 2013
- **GELDER, R.N. et al.** Amplified RNA synthesized from limited quantities of heterogeneous cDNA. *Proceedings of the National Academy of Sciences of the United States of America*, 1990, vol. 87 (5), 1663-7
- **KAWASAKI, E.S.** Microarrays and the gene expression profile of a single cell. *Annals of the New York Academy of Sciences*, 2004, vol. 1020, 92-100

- **LIVESEY, F.J**. Strategies for microarray analysis of limiting amounts of RNA. *Briefings in functional genomics & proteomics*, 2003, vol. 2 (1), 31-6
- **TANG, F.** ; **K. LAO** ; **M.A. SURANI**. Development and applications of single-cell transcriptome analysis. *Nature methods*, 2011, vol. 8, 6-11
- **HASHIMSHONY, T. et al.** CEL-Seq: single-cell RNA-Seq by multiplexed linear amplification. *Cell reports*, 2012, vol. 2 (3), 666-73
- **SHANKARANARAYANAN, P. et al.** Single-tube linear DNA amplification for genome-wide studies using a few thousand cells. *Nature protocols*, 2012, vol. 7 (2), 328-38
- **C CHEN** ; **D XING** ; **L TAN** ; **H LI** ; **G ZHOU** ; **L HUANG** ; **XS XIE**. *Science*, 2017, vol. 356 (6334), 189-194
- **C CHEN, D XING, L TAN, H LI, G ZHOU, L HUANG, XS XIE**. *Science*, 2017, vol. 356 (6334), 189-194
- **HANDYSIDE AH** ; **PENKETH RJ** ; **WINSTON RM** ; **PATTINSON JK** ; **DELHANTY JD** ; **TUDDENHAM EG**. Biopsy of human preimplantation embryos and sexing by DNA amplification. *The Lancet*, 18 February 1989, vol. 333 (8634), 347-9
- **HANDYSIDE AH** ; **KONTOGIANNI EH** ; **HARDY KR** ; **WINSTON RM**. Pregnancies from biopsied human preimplantation embryos sexed by Y-specific DNA amplification. *Nature*, 19 April 1990, vol. 344 (6268), 768-70
- **KEREM BS** ; **ROMMENS JM** ; **BUCHANAN JA** ; **MARKIEWICZ D** ; **COX TK** ; **CHAKRAVARTI A** ; **BUCHWALD M** ; **TSUI LC**. Identification of the cystic fibrosis gene: genetic analysis. *Science*, 08 September 1989, vol. 245 (4922), 1073-80
- **HANDYSIDE AH** ; **LESKO JG** ; **TARIN JJ** ; **WINSTON RM** ; **HUGHES MR**. Birth of a normal girl after in vitro fertilization and preimplantation diagnostic testing for cystic fibrosis. *New England Journal of Medicine*, 24 September 1992, vol. 327 (13), 905-9
- **LIU J** ; **LISSENS W** ; **SILBER SJ** ; **DEVROEY P** ; **LIEBAERS I** ; **VAN STEIRTEGHEM A**. Birth after preimplantation diagnosis of the cystic fibrosisΔ F508 mutation by polymerase chain reaction in human embryos resulting from intracytoplasmic sperm injection with epididymal sperm. *Jama*, 21 December 1994, vol. 272 (23), 1858-60
- **HARTON GL** ; **TSIPOURAS P** ; **SISSON ME** ; **STARR KM** ; **MAHONEY BS** ; **FUGGER EF** ; **SCHULMAN JD** ; **KILPATRICK MW** ; **LEVINSON G** ; **BLACK SH**. Preimplantation genetic testing for Marfan syndrome. *MHR: Basic science of reproductive medicine.*, 01 September 1996, vol. 2 (9), 713-5
- **AO A** ; **WELLS D** ; **HANDYSIDE AH** ; **WINSTON RM** ; **DELHANTY JD**. Preimplantation genetic diagnosis of inherited cancer: familial adenomatous polyposis coli. *Journal of assisted reproduction and genetics.*, March 1998, vol. 15, 140-4
- **SERMON K** ; **GOOSSENS V** ; **SENECA S** ; **LISSENS W** ; **DE VOS A** ; **VANDERVORST M** ; **VAN STEIRTEGHEM A** ; **LIEBAERS I**. Preimplantation diagnosis for Huntington's disease (HD): clinical application and analysis of the HD expansion in affected embryos. *Prenatal Diagnosis: Published in Affiliation With the International Society for Prenatal Diagnosis.*, December 1998, vol. 18 (13), 1427-36
- **XU K** ; **SHI ZM** ; **VEECK LL** ; **HUGHES MR** ; **ROSENWAKS Z**. First unaffected pregnancy using preimplantation genetic diagnosis for sickle cell anemia. *Jama*, 12 May 1999, vol. 281 (18), 1701-6
- **HUSSEY ND** ; **DONGGUI H** ; **FROILAND DA** ; **HUSSEY DJ** ; **HAAN EA** ; **MATTHEWS CD** ; **CRAIG JE**. Analysis of five Duchenne muscular dystrophy exons and gender determination using conventional duplex polymerase chain reaction on single cells. *Molecular human reproduction.*, 01 November 1999, vol. 5 (11), 1089-94
- **RAY PF** ; **GIGAREL N** ; **PAUL BONNEFONT J** ; **ATTIÉ T** ; **HAMAMAH S** ; **FRYDMAN N** ; **VEKEMANS M** ; **FRYDMAN R** ; **MUNNICH A**. First specific preimplantation genetic diagnosis for ornithine transcarbamylase deficiency. *Prenatal diagnosis*, December 2000, vol. 20 (13), 1048-54
- **DE RYCKE M** ; **VAN DE VELDE H** ; **SERMON K** ; **LISSENS W** ; **DE VOS A** ; **VANDERVORST M** ; **VANDERFAEILLIE A** ; **VAN STEIRTEGHEM A** ; **LIEBAERS I**. Preimplantation genetic diagnosis for sickle-cell anemia and for β-thalassemia. *Prenatal Diagnosis: Published in Affiliation With the International Society for Prenatal Diagnosis*, March 2001, vol. 21 (3), 214-22
- **MOUTOU C** ; **GARDES N** ; **RONGIERES C** ; **OHL J** ; **BETTAHAR-LEBUGLE K** ; **WITTEMER C** ; **GERLINGER P** ; **VIVILLE S**. Allele-specific amplification for preimplantation genetic diagnosis (PGD) of spinal muscular atrophy. *Prenatal diagnosis*, June 2001, vol. 21 (6), 498-503
- **VERLINSKY Y** ; **RECHITSKY S** ; **SCHOOLCRAFT W** ; **STROM C** ; **KULIEV A**. Preimplantation diagnosis for Fanconi anemia combined with HLA matching. *Jama*, 27 June 2001, vol. 285 (24), 3130-3
- **SERMON K** ; **SENECA S** ; **DE RYCKE M** ; **GOOSSENS V** ; **VAN DE VELDE H** ; **DE VOS A** ; **PLATTEAU P** ; **LISSENS W** ; **VAN STEIRTEGHEM A** ; **LIEBAERS I**. PGD in the lab for triplet repeat diseases-myotonic dystrophy, Huntington's disease and Fragile-X syndrome. *Molecular and Cellular Endocrinology.*, 22 October 2001, vol. 183, S77-85

- **GIRARDET A** ; **HAMAMAH S** ; **ANAHORY T** ; **DECHAUD H** ; **SARDA P** ; **HEDON B** ; **DEMAILLE J** ; **CLAUSTRES M**. First preimplantation genetic diagnosis of hereditary retinoblastoma using informative microsatellite markers. *MHR: Basic science of reproductive medicine*, 01 February 2003, vol. 9 (2), 111-6
- **FIORENTINO F** ; **BIRICIK A** ; **NUCCITELLI A** ; **DE PALMA R** ; **KAHRAMAN S** ; **IACOBELLI M** ; **TRENGIA V** ; **CASERTA D** ; **BONU MA** ; **BORINI A**. Strategies and clinical outcome of 250 cycles of preimplantation genetic diagnosis for single gene disorders. *Human Reproduction*, 01 March 2006, vol. 21 (3), 670-84
- **ONIZ H** ; **SLAVIN S**. Successful haematopoietic stem cell transplantation in 44 children from healthy siblings conceived after preimplantation HLA matching. *Reproductive biomedicine online*, 01 September 2014, vol. 29 (3), 340-51
- **MUNNÉ S** ; **LEE A** ; **ROSENWAKS Z** ; **GRIFO J** ; **COHEN J.** Fertilization and early embryology: Diagnosis of major chromosome aneuploidies in human preimplantation embryos. *Human reproduction*, 01 December 1993, vol. 8 (12), 2185-91
- **WILTON L** ; **WILLIAMSON R** ; **MCBAIN J** ; **EDGAR D** ; **VOULLAIRE L**. Birth of a healthy infant after preimplantation confirmation of euploidy by comparative genomic hybridization. *New England Journal of Medicine.*, 22 November 2001, vol. 345 (21), 1537-41
- **WELLS D** ; **ESCUDERO T** ; **LEVY B** ; **HIRSCHHORN K** ; **DELHANTY JD** ; **MUNNE S**. First clinical application of comparative genomic hybridization and polar body testing for preimplantation genetic diagnosis of aneuploidy. *Fertility and sterility*, 01 September 2002, vol. 78 (3), 543-9
- **TREFF NR** ; **SU J** ; **TAO X** ; **LEVY B** ; **SCOTT JR RT**. Accurate single cell 24 chromosome aneuploidy screening using whole genome amplification and single nucleotide polymorphism microarrays. *Fertility and sterility*, 01 November 2010, vol. 94 (6), 2017-21
- **GUTIÉRREZ-MATEO C** ; **COLLS P** ; **SÁNCHEZ-GARCÍA J** ; **ESCUDERO T** ; **PRATES R** ; **KETTERSON K** ; **WELLS D** ; **MUNNÉ S**. Validation of microarray comparative genomic hybridization for comprehensive chromosome analysis of embryos. *Fertility and sterility*, 01 March 2011, vol. 95 (3), 953-8
- **YANG Z** ; **LIU J** ; **COLLINS GS** ; **SALEM SA** ; **LIU X** ; **LYLE SS** ; **PECK AC** ; **SILLS ES** ; **SALEM RD**. Selection of single blastocysts for fresh transfer via standard morphology assessment alone and with array CGH for good prognosis IVF patients: results from a randomized pilot study. *Molecular cytogenetics.*, December 2012, vol. 5, 1-8

- **SCOTT JR RT** ; **UPHAM KM** ; **FORMAN EJ** ; **HONG KH** ; **SCOTT KL** ; **TAYLOR D** ; **TAO X** ; **TREFF NR**. Blastocyst biopsy with comprehensive chromosome screening and fresh embryo transfer significantly increases in vitro fertilization implantation and delivery rates: a randomized controlled trial. *Fertility and sterility.*, 01 September 2013, vol. 100 (3), 697-703
- **FORMAN EJ** ; **HONG KH** ; **FERRY KM** ; **TAO X** ; **TAYLOR D** ; **LEVY B** ; **TREFF NR** ; **SCOTT JR RT**. In vitro fertilization with single euploid blastocyst transfer: a randomized controlled trial. *Fertility and sterility.*, 01 July 2013, vol. 100 (1), 100-7
- **WELLS D** ; **KAUR K** ; **GRIFO J** ; **GLASSNER M** ; **TAYLOR JC** ; **FRAGOULI E** ; **MUNNE S**. Clinical utilisation of a rapid low-pass whole genome sequencing technique for the diagnosis of aneuploidy in human embryos prior to implantation. *Journal of medical genetics.*, 01 August 2014, vol. 51 (8), 553-62
- **RUBIO C** ; **BELLVER J** ; **RODRIGO L** ; **CASTILLÓN G** ; **GUILLÉN A** ; **VIDAL C** ; **GILES J** ; **FERRANDO M** ; **CABANILLAS S** ; **REMOHI J**. In vitro fertilization with preimplantation genetic diagnosis for aneuploidies in advanced maternal age: a randomized, controlled study. *Fertility and sterility*, 01 May 2017, vol. 107 (5), 1122-9
- **CONN CM** ; **HARPER JC** ; **WINSTON RM** ; **DELHANTY JD**. Infertile couples with Robertsonian translocations: preimplantation genetic analysis of embryos reveals chaotic cleavage divisions. *Human Genetics*, January 1998, vol. 102, 117-23
- **SCRIVEN PN** ; **HANDYSIDE AH** ; **OGILVIE CM**. Chromosome translocations: segregation modes and strategies for preimplantation genetic diagnosis. *Prenatal Diagnosis: Published in Affiliation With the International Society for Prenatal Diagnosis*, December 1998, vol. 18 (13), 1437-49
- **COONEN E** ; **MARTINI E** ; **DUMOULIN JC** ; **HOLLANDERS-CROMBACH HT** ; **DE DIESMULDERS C** ; **GERAEDTS JP** ; **HOPMAN AH** ; **EVERS JL**. Preimplantation genetic diagnosis of a reciprocal translocation t (3; 11)(q27. 3; q24. 3) in siblings. *Molecular human reproduction*, 01 March 2000, vol. 6 (3), 199-206
- **MUNNÉ S** ; **SANDALINAS M** ; **ESCUDERO T** ; **FUNG J** ; **GIANAROLI L** ; **COHEN J**. Outcome of preimplantation genetic diagnosis of translocations. *Fertility and Sterility*, 01 June 2000, vol. 73 (6), 1209-18
- **ESCUDERO T** ; **ABDELHADI I** ; **SANDALINAS M** ; **MUNNE S**. Predictive value of sperm fluorescence in situ hybridization analysis on the outcome of preimplantation genetic diagnosis for translocations. *Fertility and sterility.*, 01 June 2003, vol. 79, 1528-34

- **MELOTTE C** ; **DEBROCK S** ; **D'HOOGHE T** ; **FRYNS JP** ; **VERMEESCH JR**. Preimplantation genetic diagnosis for an insertional translocation carrier. *Human Reproduction*, 01 December 2004, vol. 19 (12), 2777-83

- **LE CAIGNEC C** ; **SPITS C** ; **SERMON K** ; **DE RYCKE M** ; **THIENPONT B** ; **DEBROCK S** ; **STAESSEN C** ; **MOREAU Y** ; **FRYNS JP** ; **VAN STEIRTEGHEM A**. Single-cell chromosomal imbalances detection by array CGH. *Nucleic acids research*, 01 January 2006, vol. 34 (9), e68

- **TRAVERSA MV** ; **CAREY L** ; **LEIGH D**. A molecular strategy for routine preimplantation genetic diagnosis in both reciprocal and Robertsonian translocation carriers. *MHR: Basic science of reproductive medicine*, 19 February 2010, vol. 16 (5), 329-37

- **FIORENTINO F** ; **KOKKALI G** ; **BIRICIK A** ; **STAVROU D** ; **ISMAILOGLU B** ; **DE PALMA R** ; **ARIZZI L** ; **HARTON G** ; **SESSA M** ; **PANTOS K**. Polymerase chain reaction-based detection of chromosomal imbalances on embryos: the evolution of preimplantation genetic diagnosis for chromosomal translocations. *Fertility and sterility*, 01 November 2010, vol. 94 (6), 2001-11

- **RIUS M** ; **OBRADORS A** ; **DAINA G** ; **RAMOS L** ; **PUJOL A** ; **MARTINEZ-PASSARELL O** ; **MARQUÈS L** ; **OLIVER-BONET M** ; **BENET J** ; **NAVARRO J**. Detection of unbalanced chromosome segregations in preimplantation genetic diagnosis of translocations by short comparative genomic hibridization. *Fertility and sterility.*, 01 July 2011, vol. 96 (1), 134-42

- **FIORENTINO F** ; **SPIZZICHINO L** ; **BONO S** ; **BIRICIK A** ; **KOKKALI G** ; **RIENZI L** ; **UBALDI FM** ; **IAMMARRONE E** ; **GORDON A** ; **PANTOS K**. PGD for reciprocal and Robertsonian translocations using array comparative genomic hybridization. *Human Reproduction*, 01 July 2011, vol. 26 (7), 1925-35

- **TREFF NR** ; **THOMPSON K** ; **RAFIZADEH M** ; **CHOW M** ; **MORRISON L** ; **TAO X** ; **GARNSEY H** ; **REDA CV** ; **METZGAR TL** ; **NEAL S**. SNP array-based analyses of unbalanced embryos as a reference to distinguish between balanced translocation carrier and normal blastocysts. *Journal of Assisted Reproduction and Genetics*, August 2016, vol. 33, 1115-9

- **ZHANG S** ; **LEI C** ; **WU J** ; **ZHOU J** ; **SUN H** ; **FU J** ; **SUN Y** ; **SUN X** ; **LU D** ; **ZHANG Y**. The establishment and application of preimplantation genetic haplotyping in embryo diagnosis for reciprocal and Robertsonian translocation carriers. *BMC medical genomics*, December 2017, vol. 10 (1), 1-9

- **TAN YQ** ; **TAN K** ; **ZHANG SP** ; **GONG F** ; **CHENG DH** ; **XIONG B** ; **LU CF** ; **TANG XC** ; **LUO KL** ; **LIN G**. Single-nucleotide polymorphism microarray-based preimplantation genetic diagnosis is likely to improve the clinical outcome for translocation carriers. *Human Reproduction.*, 01 September 2013, vol. 28 (9), 2581-92

- **CHOW JF** ; **YEUNG WS** ; **LEE VC** ; **LAU EY** ; **NG EH**. Evaluation of preimplantation genetic testing for chromosomal structural rearrangement by a commonly used next generation sequencing workflow. *European Journal of Obstetrics & Gynecology and Reproductive Biology*, 01 May 2018, vol. 224, 66-73

- **TREFF NR** ; **ECCLES J** ; **LELLO L** ; **BECHOR E** ; **HSU J** ; **PLUNKETT K** ; **ZIMMERMAN R** ; **RANA B** ; **SAMOILENKO A** ; **HSU S**. Utility and first clinical application of screening embryos for polygenic disease risk reduction. *Frontiers in endocrinology*, 04 December 2019, vol. 10, 845

- **KUMAR A** ; **IM K** ; **BANJEVIC M** ; **NG PC** ; **TUNSTALL T** ; **GARCIA G** ; **GALHARDO L** ; **SUN J** ; **SCHAEDEL ON** ; **LEVY B**. Whole-genome risk prediction of common diseases in human preimplantation embryos. *Nature medicine*, March 2022, vol. 28 (3), 513-6

- **YAN L** ; **HUANG L** ; **XU L** ; **HUANG J** ; **MA F** ; **ZHU X et al.** Live births after simultaneous avoidance of monogenic diseases and chromosome abnormality by next-generation sequencing with linkage analyses. *Proc Natl Acad Sci U S A*, 2015, vol. 112, 15964-9

- **DOKRAS A** ; **SARGENT IL** ; **ROSS C** ; **GARDNER RL** ; **BARLOW DH**. Trophectoderm biopsy in human blastocysts.. *Hum Reprod*, 1990, vol. 5, 821-825

- **KOKKALI G** ; **VRETTOU C** ; **TRAEGER-SYNODINOS J** ; **JONES GM** ; **CRAM DS** ; **STAVROU D** ; **TROUNSON AO** ; **KANAVAKIS E** ; **PANTOS K**. Birth of a healthy infant following trophectoderm biopsy from blastocysts for PGD of β-thalassaemia major: case report. *Hum Reprod*, 2005, vol. 20, 1855-1859

- **PALINI S** ; **GALLUZZI L** ; **DE STEFANI S** ; **BIANCHI M** ; **WELLS D** ; **MAGNANI M** ; **BULLETTI C.** Genomic DNA in human blastocoele fluid. *Reprod Biomed Online*, 2013, vol. 26, 603-610

- **GIANAROLI L** ; **MAGLI MC** ; **POMANTE A** ; **CRIVELLO AM** ; **CAFUERI G** ; **VALERIO M** ; **FERRARETTI AP**. Blastocentesis: a source of DNA for preimplantation genetic testing. *Results from a pilot study. Fertil Steril*, 2014, vol. 102, 1692-1699

- **TOBLER KJ** ; **ZHAO Y** ; **ROSS R** ; **BENNER AT** ; **XU X** ; **DU L** ; **BROMAN K** ; **THRIFT K** ; **BREZINA PR** ; **KEARNS WG**. Blastocoel fluid from differentiated blastocysts harbors embryonic genomic material capable of a whole-genome deoxyribonucleic acid amplification and comprehensive chromosome microarray analysis. *Fertil Steril*, 2015, vol. 104, 418-425
- **MAGLI MC** ; **POMANTE A** ; **CAFUERI G** ; **VALERIO M** ; **CRIPPA A** ; **FERRARETTI AP** ; **GIANAROLI L**. Preimplantation genetic testing: Polar bodies, blastomeres, trophectoderm cells, or blastocoelic fluid?. *Fertil Steril*, 2016, vol. 105, 676-683, 5
- **ZHANG Y** ; **LI N** ; **WANG L** ; **SUN H** ; **MA M** ; **WANG H** ; **XU X** ; **ZHANG W** ; **LIU Y** ; **CRAM DS et al.** Molecular analysis of DNA in blastocoele fluid using next-generation sequencing. *J Assist Reprod Genet*, 2016, vol. 33, 637-645
- **SHANGGUAN T** ; **HE W** ; **LI H** ; **SHANG X** ; **LIU Y** ; **BAI X** ; **LI M** ; **XIE J.** Detection and analysis of DNA material in human blastocoel fluid. *Biomed Genet Genomics*, 2017, vol. 2, 1-5
- **CAPALBO A** ; **ROMANELLI V** ; **PATASSINI C** ; **POLI M** ; **GIRARDI L** ; **GIANCANI A** ; **STOPPA M** ; **CIMADOMO D** ; **UBALDI FM** ; **RIENZI L**. Diagnostic efficacy of blastocoel fluid and spent media as sources of DNA for preimplantation genetic testing in standard clinical conditions. *Fertil Steril*, 2018, vol. 110, 870-879
- **ZHIGALINA DI** ; **JATSENKO T** ; **SKRYABIN NA** ; **KANBEKOVA OR** ; **ARTYUKHOVA VG** ; **SVETLAKOV AV** ; **TEEARU K** ; **TROŠIN A** ; **SALUMETS A** ; **KURG A**. Karyotype of the blastocoel fluid demonstrates low concordance with both trophectoderm and inner cell mass. *Fertility and sterility.*, 01 June 2018, vol. 109 (6), 1127-34
- **MAGLI MC** ; **ALBANESE C** ; **CRIPPA A** ; **TABANELLI C** ; **FERRARETTI AP** ; **GIANAROLI L**. Deoxyribonucleic acid detection in blastocoelic fluid: a new predictor of embryo ploidy and viable pregnancy. *Fertil Steril*, 2018, vol. 111, 77-85
- **GALLUZZI L** ; **PALINI S** ; **DE STEFANI S** ; **ANDREONI F** ; **PRIMITERRA M** ; **DIOTALLEVI A** ; **BULLETTI C** ; **MAGNANI M**. Extracellular embryo genomic DNA and its potential for genotyping applications. *Futur Sci OA*, 2015, vol. 1, FSO62
- **WU H** ; **DING C** ; **SHEN X** ; **WANG J** ; **LI R** ; **CAI B** ; **XU Y** ; **ZHONG Y** ; **ZHOU C**. Medium-based noninvasive preimplantation genetic diagnosis for human α-thalassemias-SEA. *Medicine (Baltimore)*, 2015, vol. 94, e669
- **XU J** ; **FANG R** ; **CHEN L** ; **CHEN D** ; **XIAO J-P** ; **YANG W** ; **WANG H** ; **SONG X** ; **MA T** ; **BO S et al.** Noninvasive chromosome screening of human embryos by genome sequencing of embryo culture medium for in vitro fertilization. *Proc Natl Acad Sci*, 2016, vol. 113, 11907-11912
- **SHAMONKI MI** ; **JIN H** ; **HAIMOWITZ Z** ; **LIU L**. Proof of concept: preimplantation genetic screening without embryo biopsy through analysis of cell-free DNA in spent embryo culture media. *Fertil Steril*, 2016, vol. 106, 1312-1318
- **FEICHTINGER M** ; **VACCARI E** ; **CARLI L** ; **WALLNER E** ; **MÄDEL U** ; **FIGL K** ; **PALINI S** ; **FEICHTINGER W**. Non-invasive preimplantation genetic screening using array comparative genomic hybridization on spent culture media: a proof-of-concept pilot study. *Reprod Biomed Online*, 2017, vol. 34, 583-589
- **LANE M** ; **ZANDER-FOX DL** ; **HAMILTON H** ; **JASPER MJ** ; **HODGSON BL** ; **FRASER M** ; **BELL F**. Ability to detect aneuploidy from cell free DNA collected from media is dependent on the stage of development of the embryo. *Fertil Steril*, 2017, vol. 108, e61
- **LIU WQ** ; **LIU JQ** ; **DU HZ** ; **LING JW** ; **SUN XF** ; **CHEN DJ**. Non-invasive preimplantation aneuploidy screening and diagnosis of beta thalassemia IVSII654 mutation using spent embryo culture medium. *Ann Med*, 2017, vol. 49, 319-328
- **HO JR** ; **ARRACH N** ; **RHODES-LONG K** ; **AHMADY A** ; **INGLES S** ; **CHUNG K** ; **BENDIKSON KA** ; **PAULSON RJ** ; **MCGINNIS LK.** Pushing the limits of detection: investigation of cell-free DNA for aneuploidy screening in embryos. *Fertil Steril*, 2018, vol. 110, 467-475, 2
- **VERA-RODRIGUEZ M** ; **DIEZ-JUAN A** ; **JIMENEZ-ALMAZAN J** ; **MARTINEZ S** ; **NAVARRO R** ; **PEINADO V** ; **MERCADER A** ; **MESEGUER M** ; **BLESA D** ; **MORENO I et al.** Origin and composition of cell-free DNA in spent medium from human embryo culture during preimplantation development.. *Obstet Gynecol Surv*, 2018, vol. 33, 745-756
- **FANG R** ; **YANG W** ; **ZHAO X** ; **XIONG F** ; **GUO C** ; **XIAO J** ; **CHEN L** ; **SONG X** ; **WANG H** ; **CHEN J et al.** Chromosome screening using culture medium of embryos fertilised in vitro: A pilot clinical study. *J Transl Med*, 2019, vol. 17, 1-8
- **HUANG L** ; **BOGALE B** ; **TANG Y** ; **LU S** ; **XIE XS** ; **RACOWSKY C**. Noninvasive preimplantation genetic testing for aneuploidy in spent medium may be more reliable than trophectoderm biopsy.. *Proc Natl Acad Sci*, 2019, vol. 116, 201907472
- **RUBIO C** ; **RIENZI L** ; **NAVARRO-SÁNCHEZ L** ; **CIMADOMO D** ; **GARCIA-PASCUAL CM** ; **ALBRICCI L** ; **SOSCIA D** ; **VALBUENA D** ; **CAPALBO A** ; **UBALDI F et al.** Embryonic cell-free DNA versus trophectoderm biopsy for aneuploidy testing: concordance rate and clinical implications. *Fertil Steril*, 2019, vol. 112, 510-519

- **YEUNG QSY** ; **ZHANG YX** ; **CHUNG JPW** ; **LUI WT** ; **KWOK YKY** ; **GUI B** ; **KONG GWS** ; **CAO Y** ; **LI TC** ; **CHOY KW**. A prospective study of non-invasive preimplantation genetic testing for aneuploidies (NiPGT-A) using next-generation sequencing (NGS) on spent culture media (SCM). *J Assist Reprod Genet*, 2019, vol. 36, 1609-1621
- **JIAO J** ; **SHI B** ; **SAGNELLI M** ; **YANG D** ; **YAO Y** ; **LIW** ; **SHAO L** ; **LU S** ; **LI D** ; **WANG X**. Minimally invasive preimplantation genetic testing using blastocyst culture medium. *Hum Reprod*, 2019, vol. 34, 1369-1379
- **OU Z** ; **DENG Y** ; **LIANG Y** ; **CHEN Z** ; **SUN L**. Improved non-invasive preimplantation genetic testing for beta-thalassemia using spent embryo culture medium containing blastocoelic fluid. *Frontiers in endocrinology.*, 20 January 2022, vol. 12, 1941
- **GALLUZZI L** ; **PALINI S** ; **DE STEFANI S** ; **ANDREONI F** ; **PRIMITERRA M** ; **DIOTALLEVI A** ; **BULLETTI C** ; **MAGNANI M.** Extracellular embryo genomic DNA and its potential for genotyping applications. *Futur Sci OA*, 2015, vol. 1, FSO62
- **CHAN KA** ; **ZHANG J** ; **HUI AB** ; **WONG N** ; **LAU TK** ; **LEUNG TN** ; **LO KW** ; **HUANG DW** ; **LO YD**. Size distributions of maternal and fetal DNA in maternal plasma. *Clinical chemistry*, 01 January 2004, vol. 50 (1), 88-92
- **HUANG L** ; **MA F** ; **CHAPMAN A** ; **LU S** ; **XIE XS**. Single-cell whole-genome amplification and sequencing: methodology and applications. *Annual review of genomics and human genetics.*, 24 August 2015, vol. 16, 79-102
- **CHEN C** ; **XING D** ; **TAN L** ; **LI H** ; **ZHOU G** ; **HUANG L** ; **XIE XS**. Single-cell whole-genome analyses by Linear Amplification via Transposon Insertion (LIANTI). *Science*, 14 April 2017, vol. 356 (6334), 189-94
- **LEAVER M** ; **WELLS D**. Non-invasive preimplantation genetic testing (niPGT): the next revolution in reproductive genetics?. *Human reproduction update.*, 01 January 2020, vol. 26 (1), 16-42
- **CIMADOMO D** ; **RIENZI L** ; **CAPALBO A** ; **RUBIO C** ; **INNOCENTI F** ; **GARCIA-PASCUAL CM** ; **UBALDI FM** ; **HANDYSIDE A**. The dawn of the future: 30 years from the first biopsy of a human embryo. The detailed history of an ongoing revolution. *Human Reproduction Update.*, July 2020, vol. 26 (4), 453-73
- **DE RYCKE M** ; **GOOSSENS V.** ; **KOKKALI G.** ; **MEIJER-HOOGEVEEN M** ; **COONEN E** ; **MOUTOU C.** ESHRE PGD Consortium data collection XIV-XV: Cycles from. *Hum. Reprod. 2017*, January 2011, vol. 32, 1974-1994
- **ESHRE PGT-M WORKING GROUP** ; **CARVALHO F** ; **MOUTOU C** ; **DIMITRIADOU E** ; **DREESEN J** ; **GIMÉNEZ C** ; **GOOSSENS V** ; **KAKOUROU G** ; **VERMEULEN N** ; **ZUCCARELLO D**. ESHRE PGT Consortium good practice recommendations for the detection of monogenic disorders. *Human reproduction open*, 2020 (3), 1-18
- **XUE, A. et al.** Genome-wide association analyses identify 143 risk variants and putative regulatory mechanisms for type 2 diabetes. *Nat. Commun*, 2018, vol. 9, 2941
- **FAN, H. C.** ; **GU, W** ; **WANG, J.** ; **BLUMENFELD, Y. J** ; **EL-SAYED, Y. Y** ; **QUAKE, S. R**. Non-invasive prenatal measurement of the fetal genome. *Nature*, 2012, vol. 487 (7407), 320-324
- **NABIEVA, E** ; **SHARMA, S M** ; **KAPUSHEV Y et al.** Accurate fetal variant calling in the presence of maternal cell contamination. *European Journal of Human Genetics*, 2020, vol. 28 (11), 1615-1623
- **LANDER, E.S.** ; **GREEN, P**. Construction of multilocus genetic linkage maps in humans. *Proceedings of the National Academy of Sciences*, 1987, vol. 84 (8), 2363-2367
- **KRUGLYAK L** ; **DALY M J** ; **REEVE-DALY M P et al.** Parametric and nonparametric linkage analysis: a unified multipoint approach. *American journal of human genetics*, 1996, vol. 58 (6), 1347
- **IDURY R M** ; **ELSTON R C**. A faster and more general hidden Markov model algorithm for multipoint likelihood calculations. *Human heredity*, 1997, vol. 47 (4), 197-202
- **KRUGLYAK L** ; **LANDER E S**. Faster multipoint linkage analysis using Fourier transforms. *J. Comput. Biol.*, 1998, vol. 5 (1), 1-7
- **ABECASIS G R** ; **CHERNY S S** ; **COOKSON W O et al.** Merlin-rapid analysis of dense genetic maps using sparse gene flow trees. *Nature genetics*, 2002, vol. 30 (1), 97-101